# EUROPEAN PATENT APPLICATION

(11) **EP 4 793 718 A1**
(43) Date of publication of application: **19.08.2026**
(21) Application number: 24894331.8
(22) Date of filing: 06.09.2024
(51) Int. Cl.: G04B 37/14, G04G 17/08, H01F 7/122, G09F 9/30, G01L 1/14, G01C 17/30, G06F 1/16

(54) **WEARABLE ELECTRONIC DEVICE INCLUDING ELECTRONIC MAGNET**

(30) Priority: 20.11.2023 KR 20230160854; 15.12.2023 KR 20230183436
(71) Applicant: Samsung Electronics Co., Ltd., Suwon-si, Gyeonggi-do 16677 (KR)
(72) Inventor: KIM, Taesan, Suwon-si Gyeonggi-do 16677 (KR); MOON, Shinhun, Suwon-si Gyeonggi-do 16677 (KR); CHOI, Yongjun, Suwon-si Gyeonggi-do 16677 (KR)
(74) Representative: Appleyard Lees IP LLP
(86) International application number: PCT/KR2024/013529
(87) International publication number: WO 2025/110435

(57) **Abstract**

According to an embodiment, a wearable electronic device may comprise: a housing; a first wearable member detachably disposed on one side of the housing; a second wearable member that is detachably disposed on the other side of the housing and aligned with the first wearable member along a first direction with the housing therebetween, wherein the second wearable member can be coupled to at least partially face the first wearable member and thereby form a closed curve together with the housing and the first wearable member; first magnetic bodies which are arranged in the first wearable member so that first polarities and second polarities are alternately arranged along the first direction; and at least one pair of second magnetic bodies arranged in the second wearable member along the first direction. In one embodiment, the at least one pair of second magnetic bodies may be configured to receive electrical signals and generate attractive or repulsive force with respect to the first magnetic bodies. Various other embodiments may also be possible.

## Description

### [Technical Field]

One or more embodiments of the disclosure relates to an electronic device such as a wearable electronic device including an electromagnet.

### [Background Art]

Typically, the term "electronic device" may refer to a device that performs a specific function based on an installed program, such as a home appliance, an electronic scheduler, a portable multimedia player, a mobile communication terminal, a tablet PC, a video/audio device, a desktop/laptop PC, or a vehicle navigation system. As the integration level of electronic devices increases and ultra-high-speed, large-capacity wireless communications become widespread, various functions may be incorporated into a single compact wearable electronic device, such as a mobile communication terminal. For example, not only communication functions but also entertainment functions such as gaming, multimedia functions such as music and video playback, communication and security functions for mobile banking, as well as schedule management and electronic wallet functions, are being integrated into a single electronic device.

Recently, wearable electronic devices, which are wearable on a body, have been commercialized, and mobile communication terminals and wearable electronic devices are being used daily. Since a wearable electronic device may be maintained for a considerable amount of time in the state of being in contact with a user's body, the electronic device may be useful for medical or health care. For example, depending on sensors mounted thereon, an electronic device may detect biometric information such as a user's photoplethysmograph (PPG), sleep intervals, skin temperature, heart rate, and/or electrocardiogram, and the detected biometric information may be stored in the electronic device or transmitted in real time to a medical institution for use in managing the user's health. In general, electronic devices have a bar shape, a box shape, or a flat plate shape; however, wearable electronic devices may include a combination of a plurality of segments in consideration of wearing convenience while accommodating curvature of a user's body. For example, a wrist-worn electronic device may include a housing that accommodates various circuit devices and serves as a main body, and at least one wearing member, and a face-worn electronic device may include one or more lenses corresponding to both eyes of a user and at least one temple bow.

The above-described information may be provided as related art for the purpose of helping to understand the disclosure. No claim or determination is made regarding whether any of the foregoing may be applied as prior art with respect to the disclosure.

### [Detailed Description of the Invention]

### [Technical Solution]

According to an embodiment of the disclosure, a wearable electronic device may include a housing, a first wearing member detachably disposed on one side of the housing, and a second wearing member detachably disposed on the other side of the housing and aligned with the first wearing member along a first direction with the housing interposed therebetween. The second wearing member may be configured to be coupled so as to at least partially face the first wearing member, thereby forming a closed loop together with the housing and the first wearing member. The wearable electronic device may further include first magnetic bodies disposed on the first wearing member such that first polarities and second polarities are alternately arranged along the first direction, and at least one pair of second magnetic bodies arranged on the second wearing member along the first direction. According to an embodiment, the at least one pair of second magnetic bodies may be configured to generate an attractive force or a repulsive force with the first magnetic bodies in response to receiving an electrical signal.

According to an embodiment of the disclosure, a wearable electronic device may include a housing, a first wearing member detachably disposed on one side of the housing, and a second wearing member detachably disposed on the other side of the housing and aligned with the first wearing member along a first direction with the housing interposed therebetween. The second wearing member may be configured to be coupled so as to at least partially face the first wearing member, thereby forming a closed loop together with the housing and the first wearing member. The wearable electronic device may further include permanent magnets disposed on the first wearing member such that first polarities and second polarities are alternately arranged along the first direction, and at least one pair of electromagnets arranged on the second wearing member along the first direction. According to an embodiment, the at least one pair of electromagnets may be configured to generate an attractive force or a repulsive force with the permanent magnets in response to receiving an electrical signal.

According to an embodiment of the disclosure, a wearable electronic device may include a housing, a first wearing member detachably disposed on one side of the housing, and a second wearing member detachably disposed on the other side of the housing and aligned with the first wearing member along a first direction with the housing interposed therebetween. The second wearing member may be configured to be coupled so as to at least partially face the first wearing member, thereby forming a closed loop together with the housing and the first wearing member. The wearable electronic device may further include permanent magnets disposed on the first wearing member, and at least one pair of electromagnets arranged on the second wearing member along the first direction. In an embodiment, the at least one pair of electromagnets may be configured to receive an electrical signal and generate an attractive force or a repulsive force with the permanent magnets, thereby moving the first wearing member and the second wearing member relative to each other in the first direction or in a second direction opposite to the first direction.

### [Brief Description of Drawings]

The above-described aspects or other aspects, configurations, and/or advantages regarding an embodiment of the disclosure may become more apparent through the following detailed description made with reference to the accompanying drawings.
FIG. 1 is a block diagram illustrating an electronic device according to an embodiment of the disclosure within a network environment.
FIG. 2 is a front perspective view illustrating a wearable electronic device according to an embodiment of the disclosure.
FIG. 3 is a rear perspective view illustrating the wearable electronic device of FIG. 1 according to an embodiment of the disclosure.
FIG. 4 is an exploded perspective view illustrating the wearable electronic device of FIG. 1 according to an embodiment of the disclosure.
FIG. 5 is a perspective view illustrating a first wearing member of a wearable electronic device according to an embodiment of the disclosure.
FIG. 6 is an enlarged perspective view illustrating a portion of the first wearing member of the wearable electronic device according to an embodiment of the disclosure.
FIG. 7 is an enlarged perspective view illustrating a portion of the second wearing member of the wearable electronic device according to an embodiment of the disclosure.
FIG. 8 is a view illustrating a first wearing state of the wearable electronic device according to an embodiment of the disclosure.
FIG. 9 is a view illustrating a first wearing state of a wearable electronic device according to an embodiment of the disclosure.
FIG. 10 is a view illustrating an operation of changing a wearing state of a wearable electronic device according to an embodiment of the disclosure.
FIG. 11 is a view illustrating a second wearing state of the wearable electronic device according to an embodiment of the disclosure.
FIG. 12 is a view illustrating a second wearing state of a wearable electronic device according to an embodiment of the disclosure.
FIG. 13 is a view illustrating a third wearing state of the wearable electronic device according to an embodiment of the disclosure.
FIG. 14 is a block diagram of a wearable electronic device according to an embodiment of the disclosure.
FIG. 15 is a view illustrating an operation of entering a wearing state according to an exercise-related function in a wearable electronic device according to an embodiment of the disclosure.
FIG. 16 is a view illustrating an operation of entering a wearing state according to an exercise-related function in a wearable electronic device according to an embodiment of the disclosure.
FIG. 17 is a flowchart illustrating operations of entering wearing states according to an exercise-related function in a wearable electronic device according to an embodiment of the disclosure.

Throughout the appended drawings, like reference numerals may be assigned to like components, configurations, and/or structures.

### [Mode for Carrying out the Invention]

When a wearable electronic device is used for medical or healthcare purposes, for example, for detecting user biometric information, the accuracy of the detected biometric information may increase as the wearable electronic device is brought into closer contact with a user's body. Although wearable electronic devices are continuously being reduced in size and weight for comfortable wearing, a wearing state for detecting biometric information in which a wearable electronic device is in close contact with a user's body may degrade wearing comfort. Wearable electronic devices may also be useful in measuring an amount of exercise, a travel distance, and/or an exercise intensity. In such exercise modes, more accurate biometric information or exercise-related data may be detected by bringing the wearable electronic device into close contact with the user's body. However, when the wearable electronic device is in close contact with the user's body, bodily secretions (e.g., sweat) may cause user injuries, such as skin damage.

An embodiment of the disclosure is directed to at least alleviating the above-described problems and/or disadvantages and to at least provide the advantages described below, and may provide a wearable electronic device including electromagnets, thereby facilitating adjustment of a wearing state.

An embodiment of the disclosure may provide a wearable electronic device that offers wearing comfort during normal times while improving accuracy of biometric information detection in an exercise mode.

An embodiment of the disclosure may provide a wearable electronic device that is capable of suppressing contamination or user injuries caused by bodily secretions.

An embodiment of the disclosure may provide a wearable electronic device that offers a wearing state optimized for a user in each of a state in which an exercise-related function is not executed, a state in which the exercise-related function is executed, and a state in which execution of the exercise-related function is terminated.

The technical issues to be addressed by the disclosure are not limited to those described above, and other technical issues may be clearly understood by a person ordinarily skilled in the related art to which the disclosure pertains.

The following description with reference to the appended drawings may provide an understanding of various exemplary implementations of the disclosure, including the claims and their equivalents. An exemplary embodiment disclosed in the following description includes various specific details to help understanding, but is considered to be one of various exemplary embodiments. Accordingly, it will be understood by a person ordinarily skilled in the art that various implementations described herein may be variously changed and modified without departing from the scope and spirit of the disclosure. In addition, descriptions of well-known functions and configurations may be omitted for clarity and conciseness.

The terms and words used in the following description and claims are not limited to a bibliographical meaning, but may be used to describe an embodiment of the disclosure clearly and consistently. Accordingly, it will be apparent to those skilled in the art that the following description of various implementations of the disclosure is provided for explanatory purposes and not intended to limit the scope of the disclosure and equivalents thereof.

Unless the context clearly indicates otherwise, the singular forms of "a," "an," and "the" should be understood to include the plural meaning. Accordingly, for example, the term "a component surface" may be understood to include one or more surfaces of the component.

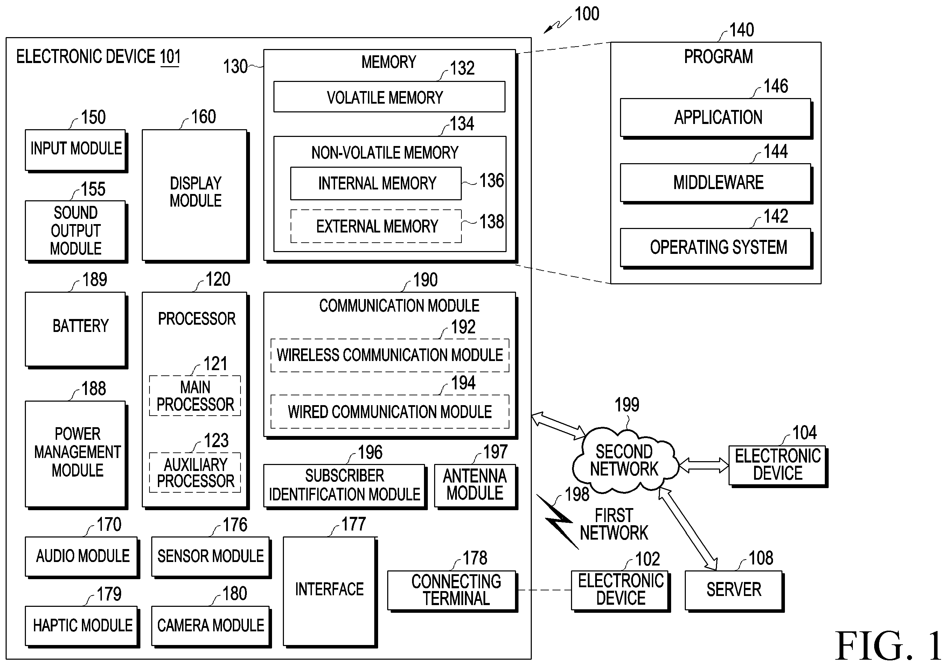

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{TM}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program) including one or more instructions that are stored in a storage medium (e.g., internal memory or external memory) that is readable by a machine (e.g., the electronic device). For example, a processor (e.g., the processor) of the machine (e.g., the electronic device) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities, and some of the multiple entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to an embodiment, operations performed by the module, the program, or another component may be carried out sequentially, in parallel, repeatedly, or heuristically, or one or more of the operations may be executed in a different order or omitted, or one or more other operations may be added.

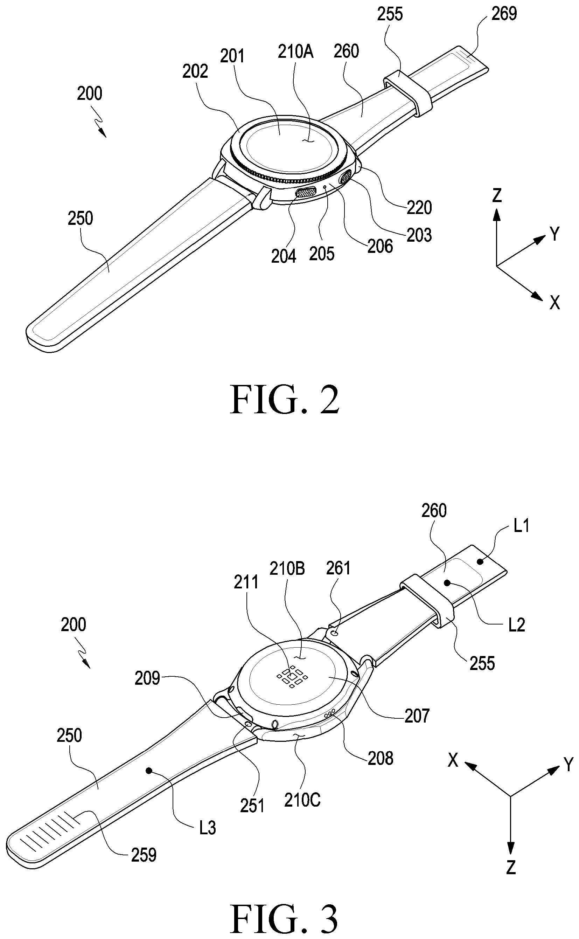

In the following detailed description, a length direction, a width direction, and/or a thickness direction of an electronic device (e.g., the wearable electronic device 200 or 300 of FIGS. 2 to 4) may be referred to, and the length direction may be defined as a "Y-axis direction," the width direction may be defined as an "X-axis direction," and/or the thickness direction may be defined as a "Z-axis direction." In an embodiment, the direction in which a component is oriented may be mentioned along with the orthogonal coordinate system illustrated in the drawings, as well as indications of "negative/positive symbol (-/+)" directions For example, the front side of an electronic device and/or a housing may be defined as a "side oriented in the +Z direction," and the rear side may be defined as a "side oriented in the -Z direction." In an embodiment, a lateral side of an electronic device and/or a housing may include an area oriented in the +X direction, an area oriented in the +Y direction, an area oriented in the -X direction, and/or an area oriented in the -Y direction. In an embodiment, the "X-axis direction" may include both the "-X direction" and the "+X direction." It is noted that these are based on the Cartesian coordinate system illustrated in the drawings for the sake of brevity of description, and the descriptions of these directions or components do not limit one or more embodiments of the disclosure. For example, depending on the design specifications of an electronic device or a user's usage habits, the Cartesian coordinate system may be defined differently from that disclosed therein.

FIG. 2 is a front perspective view illustrating a wearable electronic device 200 according to an embodiment of the disclosure. FIG. 3 is a rear perspective view illustrating the wearable electronic device 200 of FIG. 2 according to an embodiment of the disclosure.

Referring to FIGS. 2 and 3, the wearable electronic device 200 according to an embodiment may include a housing 210 including a first surface (or front surface) 210A, a second surface (or rear surface) 210B, and a side surface 210C surrounding a space between the first surface 210A and the second surface 210B, and wearing members 250 and 260 each connected to at least a portion of the housing 210 and configured to detachably fasten the electronic device 200 to a part of a user's body (e.g., a wrist or an ankle). For example, the wearable electronic device 200 may take the form of a wristwatch. In an embodiment (not illustrated), the housing may refer to a structure that forms a portion of the first surface 210A of FIG. 2, and the second surface 210B and the side surface 210C of FIG. 3. According to an embodiment, at least a portion of the first surface 210A may be defined by a substantially transparent front surface plate 201 (e.g., a glass plate or a polymer plate including various coating layers). The second surface 210B may be made of a substantially opaque rear surface plate 207. In an embodiment, when the electronic device includes a sensor module 211 disposed on the second surface 210B, the rear surface plate 207 may include an at least partially transparent area. The rear surface plate 207 may be made of, for example, coated or colored glass, ceramic, polymer, metal (e.g., aluminum, stainless steel (STS), or magnesium), or a combination of two or more of these materials. The side surface 210C may be defined by a side surface bezel structure (or a "side member") 206 coupled to the front surface plate 201 and the rear surface plate 207 and including metal and/or polymer. In an embodiment, the rear surface plate 207 and the side bezel structure 206 may be integrally configured and may include the same material (e.g., a metal material such as aluminum). The wearing members 250 and 260 may be formed of various materials and in various shapes. Multiple integrated unit links may be disposed to be movable with respect to each other by using a woven material, leather, rubber, urethane, metal, ceramic, or a combination of two or more of these materials.

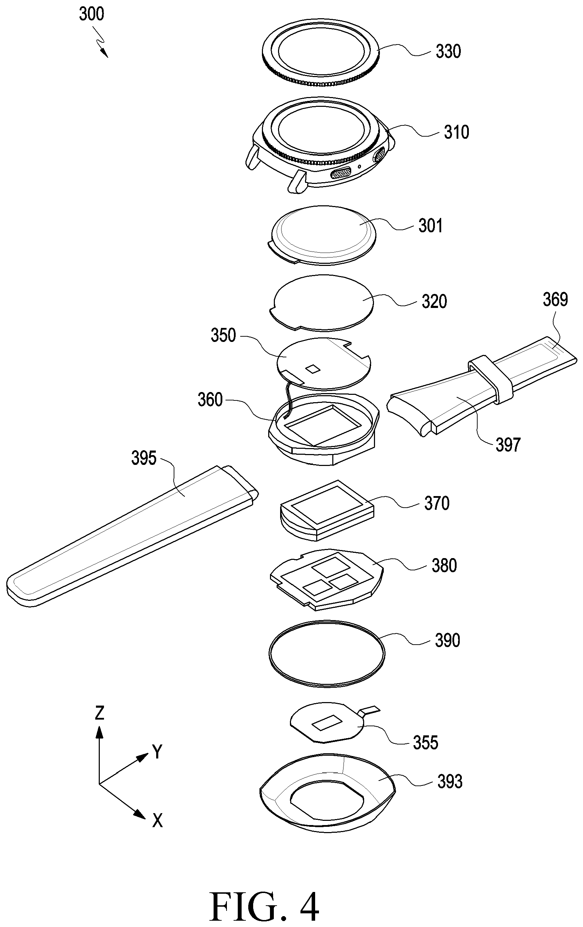

According to an embodiment, the electronic device 200 may include at least one of a display 320 (see FIG. 4), audio modules 205 and 208, a sensor module 211, key input devices 202, 203, and 204, and a connector hole 209. In an embodiments, in the electronic device 200, at least one of the components (e.g., the key input devices 202, 203, and 204, the connector hole 209, or the sensor module 211) may be omitted or other components may be additionally included.

The display (e.g., the display 320 in FIG. 4) may be exposed, for example, through a substantial portion of the front surface plate 201. The display 320 may have a shape corresponding to the shape of the front surface plate 201, and may have various shapes such as a circle, an ellipse, and a polygon. The display 320 may be coupled to or disposed adjacent to a touch detection circuit, a pressure sensor capable of measuring touch intensity (pressure), and/or a fingerprint sensor.

The audio modules 205 and 208 may include a microphone hole 205 and a speaker hole 208. A microphone configured to acquire external sound may be disposed inside the microphone hole 205, and in an embodiment, multiple microphones may be disposed to detect the direction of sound. The speaker hole 208 may be used for an external speaker and a call receiver. In an embodiment, a speaker may be included without a speaker hole (e.g., a piezo speaker).

The sensor module 211 may generate electrical signals or data values corresponding to an internal operating state or an external environmental state of the electronic device 200. The sensor module 211 may include, for example, a biometric sensor module 211 (e.g., an HRM sensor) disposed on the second surface 210B of the housing 210. The electronic device 200 may further include at least one of sensor modules (not illustrated), such as a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 203, and 204 may include a wheel key 202 disposed on the first surface 210A of the housing 210 and configured to be rotatable in at least one direction, and/or side key buttons 203 and 204 disposed on the side surface 210C of the housing 210. The wheel key 202 may have a shape corresponding to the shape of the front surface plate 201. In an embodiment, the electronic device 200 may not include some or all of the above-mentioned key input devices 202, 203, and 204, and the key input devices 202, 203, and 204, which are not included, may be implemented in other forms such as soft keys on the display 320. The connector hole 209 may accommodate a connector (e.g., a USB connector) configured to transmit and receive power and/or data to and from an external electronic device and may include another connector hole (not illustrated) configured to accommodate a connector for transmitting and receiving audio signals to and from an external electronic device. The electronic device 200 may further include, for example, a connector cover (not illustrated), which covers at least a portion of the connector hole 209 and blocks inflow of external foreign matter into the connector hole.

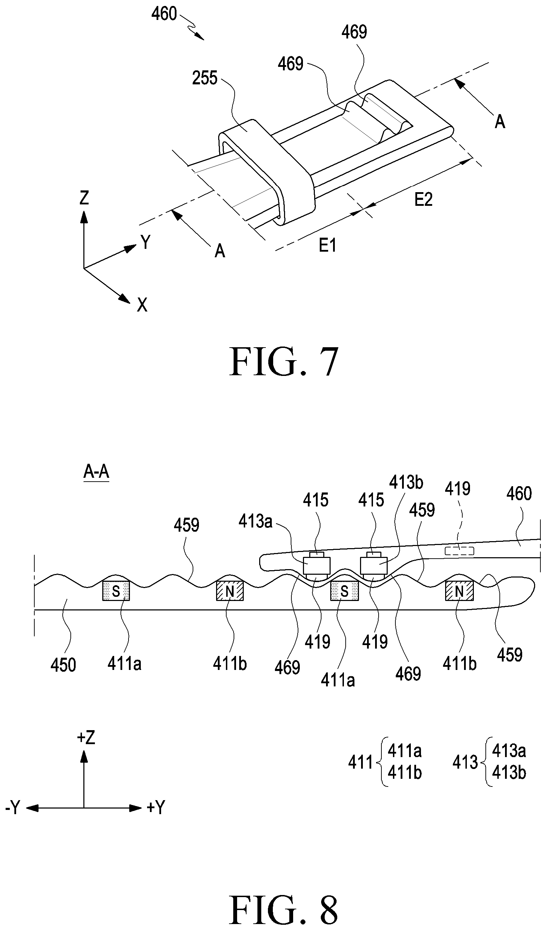

Each of the wearing members 250 and 260 may be detachably coupled to at least a portion of the housing 210 by using a locking member 251 or 261. The locking members 251 and 261 may include binding components such as pogo pins, and depending on an embodiment, the locking members may be replaced with protrusions or grooves provided on the wearing members 250 and 260. For example, the wearing members 250 and 260 may be coupled to the housing 210 by engaging with the grooves or protrusions provided on the housing 210. According to an embodiment, the wearing members 250 and 260 may be coupled to each other in a state of at least partially facing each other by including fastening structures 259 and 269, or by including at least one permanent magnet (e.g., the permanent magnet 411 of FIG. 8) or at least one metal piece (e.g., the metal piece 419 of FIG. 8) to be described later. Here, the metal piece may refer to a piece made of a material that is magnetized adjacent to a magnetic field or within a magnetic field. In an embodiment, the wearing members 250 and 260 may be coupled so as to partially face each other, thereby forming a substantially closed loop together with the housing 210. For example, the wearable electronic device 200 may be worn on a user's wrist in a state in which the housing 210 and/or the wearing members 250 and 260 surround a part of the user's body (e.g., a wrist).

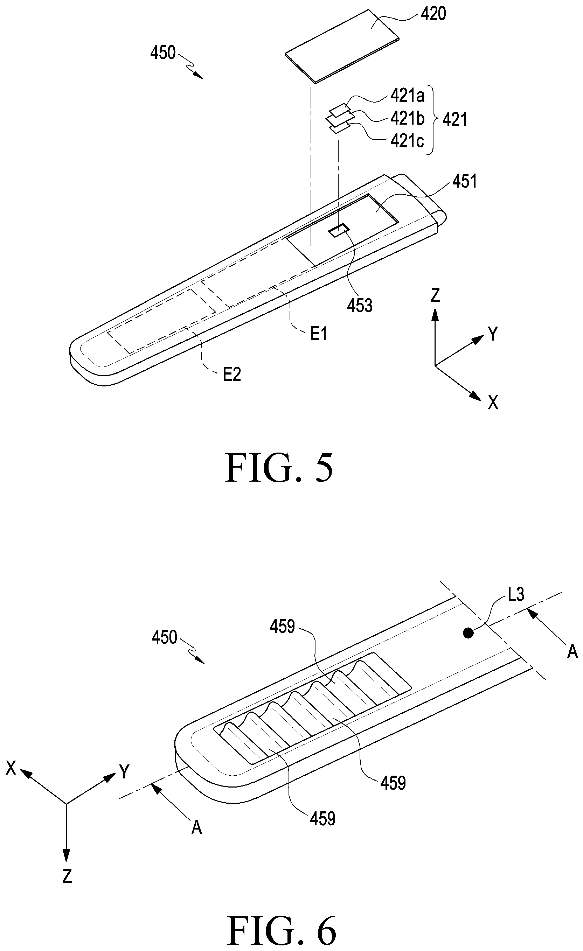

According to an embodiment, of the fastening structures 259 and 269, a first fastening structure 259 may be provided on an inner surface of the first wearing member indicated by reference numeral "250," and a second fastening structure 269 may be provided on an outer surface of the second wearing member indicated by reference numeral "260." For example, the wearing members 250 and 260 may be coupled in a state in which the first wearing member faces the outer surface of the second wearing member. According to an embodiment, a coupled state of the wearing members 250 and 260 may be stably maintained by a combination of the fastening structures 259 and 269 and/or at least one permanent magnet and at least one metal piece embedded in the wearing members 250 and 260. The wearing members 250 and 260 and/or the fastening structures 259 and 269 will be described again with reference to FIGS. 5 to 7.

According to an embodiment, the wearable electronic device 200 and/or the wearing members 250 and 260 may further include a guide member 255. According to an embodiment, the wearing members 250 and 260 may maintain a state of being in close contact with each other at portions where the fastening structures 259 and 269 are disposed. According to an embodiment, the first wearing member indicated by reference numeral "250" may be disposed so as to wrap a portion of an outer surface of the second wearing member indicated by reference numeral "260," and the guide member 255 may maintain the first wearing member 250 in a state of being substantially in contact with the second wearing member 260.

According to an embodiment, the wearable electronic device 200 may include a sensor (e.g., the pressure sensor 321 of FIG. 5) configured to detect a state in which the wearable electronic device 200 is worn on a user's body. Such a pressure sensor may be disposed at, for example, at least one of positions indicated by "L1," "L2," and/or "L3" of FIG. 3. However, in one or more embodiments of the disclosure, the number and positions of sensors embedded in the wearing members 250 and 260 are not limited to those illustrated in the drawings and may be appropriately changed according to manufacturing specifications of the wearable electronic device 200. According to an embodiment, the wearable electronic device 200, for example, memory (e.g., the memory 130 of FIG. 1), may include a table related to pressure data for a first wearing state indicating a comfortable wearing state (e.g., a first predetermined range for a first pressure) and/or pressure data for a second wearing state indicating a state in which biometric information measurement is facilitated (e.g., a second predetermined range for a second pressure). According to an embodiment, a processor (e.g., the processor 120 of FIG. 1) may execute instructions stored in the memory to detect pressure using a sensor and determine a wearing state of the wearable electronic device 200 based on the detected pressure and/or the data table stored in the memory.

FIG. 4 is an exploded perspective view illustrating the wearable electronic device of FIG. 2 according to an embodiment of the disclosure.

Referring to FIG. 4, the wearable electronic device 300 may include a side surface bezel structure 310, a wheel key 330, a front surface plate 301 (e.g., the front surface plate 201 of FIG. 2), a display 320, a first antenna 350, a second antenna (e.g., an antenna included in the second circuit board 355), a support member 360 (e.g., a bracket), a battery 370, a printed circuit board 380, a sealing member 390, a rear surface plate 393, and wearing members 395 and 397 (e.g., the wearing members 250 and 260 of FIG. 2 or FIG. 3). The wearing members 395 and 397 may include a fastening structure 369 (e.g., the fastening structures 259 and 269 of FIG. 2 or FIG. 3), thereby being coupled so as to at least partially face each other to implement a closed-loop structure together with a housing (e.g., the side bezel structure 310). At least one of the components of the electronic device 300 may be the same as or similar to at least one of the components of the electronic device 200 illustrated in FIG. 2 or FIG. 3, and redundant descriptions thereof are omitted below. The support member 360 may be disposed inside the electronic device 300 and connected to the side bezel structure 310, or may be integrally configured with the side bezel structure 310. The support member 360 may be made of, for example, a metal material and/or a non-metal (e.g., polymer) material. The support member 360 may include one surface to which the display 320 is coupled and the other surface to which the printed circuit board 380 is coupled. The printed circuit board 380 may be equipped with a processor, memory, and/or an interface. The processor may include, for example, one or more of a central processing unit, an application processor, a graphics processing unit (GPU), an application processor sensor processor, or a communication processor.

The memory may include, for example, volatile memory or non-volatile memory. The interface may include, for example, a high-definition multimedia interface (HDMI), a universal serial bus (USB) interface, an SD card interface, and/or an audio interface. The interface may electrically or physically connect, for example, the electronic device 300 to an external electronic device, and may include a USB connector, an SD card/MMC connector, or an audio connector.

The battery 370 may serve as a device that supplies power to at least one component of the electronic device 300, and may include, for example, a non-rechargeable primary cell, a rechargeable secondary cell, or a fuel cell. At least a portion of the battery 370 may be, for example, disposed on substantially the same plane as the printed circuit board 380. The battery 370 may be integrally disposed inside the electronic device 300, or may be detachably disposed on the electronic device 300.

The first antenna 350 may be disposed between the display 320 and the support member 360. The first antenna 350 may include, for example, a near-field communication (NFC) antenna, a wireless charging antenna, and/or a magnetic secure transmission (MST) antenna. For example, the first antenna 350 may perform short-range communication with an external device, may wirelessly transmit/receive power required for charging, or may transmit a short-range communication signal or a magnet-based signal including payment data. In an embodiment, an antenna structure may be formed by a portion or a combination of the side bezel structure 310 and/or the support member 360.

The second circuit 355 may be disposed between the printed circuit board 380 and the rear surface plate 393. The second circuit board 355 may include an antenna, for example, a near-field communication (NFC) antenna, a wireless charging antenna, and/or a magnetic secure transmission (MST) antenna. For example, the second circuit board 355 may perform short-range communication with an external device, may wirelessly transmit/receive power required for charging, or may transmit a short-range communication signal or a magnet-based signal including payment data. In an embodiment, an antenna structure may be formed by a portion or a combination of the side bezel structure 310 and/or the rear surface plate 393. In various embodiments, when the electronic device 300 (e.g., the electronic device 200 of FIGS. 2 and 3) includes a sensor module (e.g., the sensor module 211 of FIG. 3), a sensor circuit disposed on the second circuit board 355 or a sensor element separate from the second circuit board 355 (e.g., a photoelectric conversion element or an electrode pad) may be disposed. For example, an electronic component provided as the sensor module 211 may be disposed between the circuit board 380 and the rear surface plate 393.

The sealing member 390 may be located between the side bezel structure 310 and the rear surface plate 393. The sealing member 390 may be configured to block moisture and foreign matter from flowing into the space surrounded by the side bezel structure 310 and the rear surface plate 393 from the outside.

FIG. 5 is a perspective view illustrating a first wearing member 450 (e.g., the wearing member indicated by reference numeral "250" of FIG. 2 or FIG. 3) of a wearable electronic device (e.g., the wearable electronic device 200 or 300 of FIGS. 2 to 4) according to an embodiment of the disclosure. FIG. 6 is an enlarged perspective view illustrating a portion of the first wearing member 450 of the wearable electronic device 300 according to an embodiment of the disclosure.

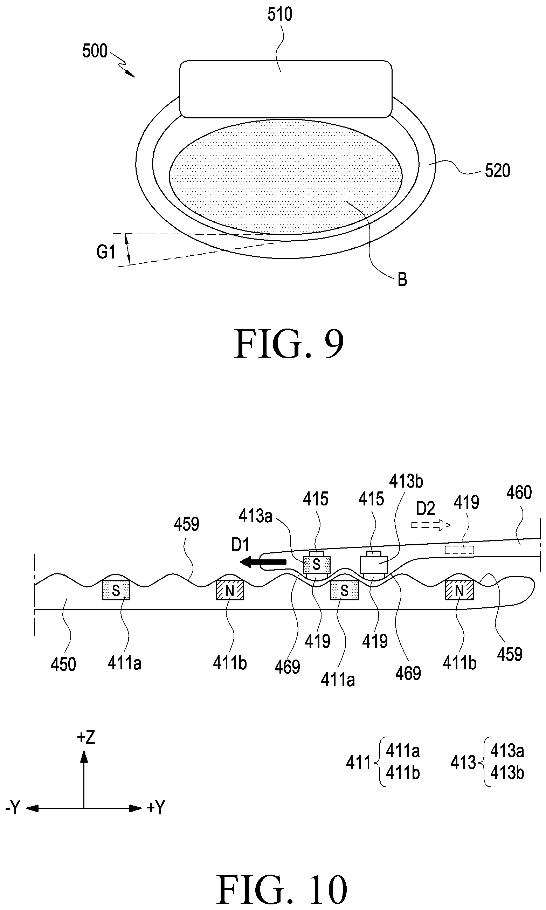

Referring to FIGS. 5 and 6, the first wearing member 450 may be fastened to a housing (e.g., the housing 210 of FIG. 2) by a fastening component (e.g., the locking member 251 of FIG. 3). According to an embodiment, the housing 210 and the first wearing member 450 (and/or the second wearing member 460 of FIG. 7) may be understood as being aligned along a length direction of the wearable electronic device 300 (e.g., the Y-axis direction, or the first direction D1 and/or the second direction D2 of FIG. 10). For example, the first wearing member 450 may be detachably disposed on one side of the housing 210, and the second wearing member 460 may be detachably disposed on the other side of the housing 210.

According to an embodiment, the wearable electronic device 300 and/or the first wearing member 450 may include at least one of a flexible display 420, a pressure sensor 421, and/or valley-shaped portions 459 (e.g., the first fastening structure 259 of FIG. 3). According to an embodiment, for arrangement of the flexible display 420 and/or the pressure sensor 421, the wearable electronic device 300 and/or the first wearing member 450 may further include accommodation grooves 451 and 453.

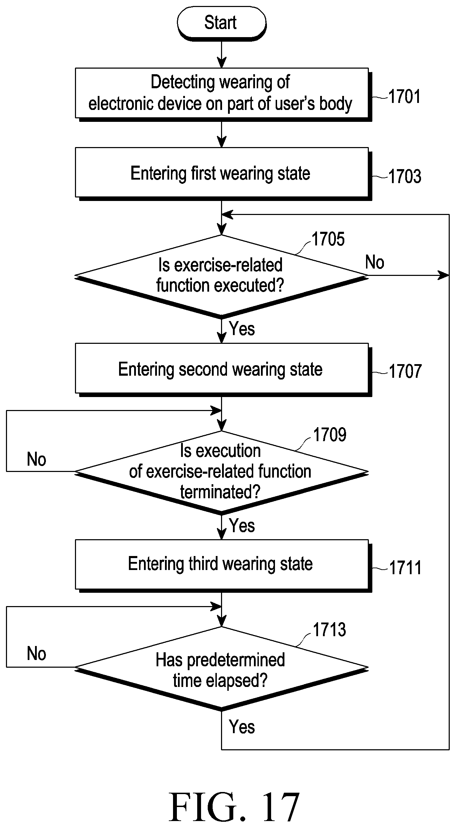

According to an embodiment, the accommodation grooves 451 and 453 may be provided on an outer surface of the first wearing member 450 (e.g., the surface oriented in the +Z direction in FIG. 5). For example, when the wearable electronic device 300 is worn on a user's body, the flexible display 420 may be substantially visually exposed to an external space. The accommodation grooves 451 and 453 may include, for example, a first accommodation groove 451 configured to accommodate the flexible display 420 and a second accommodation groove 453 configured to accommodate the pressure sensor 421. In the illustrated embodiment, the first accommodation groove 451 may be understood as having a recessed shape from a surface of the first wearing member 450, and/or the second accommodation groove 453 may be understood as having a recessed shape from a bottom surface of the first accommodation groove 451. For example, the pressure sensor 421 may be disposed so as to overlap the flexible display 420. However, one or more embodiments of the disclosure are not limited thereto, and the second accommodation groove 453 may be provided at a position independent from the first accommodation groove 451, for example, at a position indicated by "L3" of FIG. 6. In an embodiment, the pressure sensor 421 may be understood as being disposed in an area of the first wearing member 450 or the second wearing member 460 that comes into direct contact with a user's skin. For example, when the wearable electronic device 300 is worn on a user's body, the pressure sensor 421 may be disposed at a position suitable for detecting a pressure (or a change in the pressure) according to one or more wearing states to be described later with reference to FIG. 17. As described above, the pressure sensor 421 may be disposed on at least one of the first wearing member 450, the second wearing member 460, and/or the housing 210 in consideration of manufacturing specifications of the wearable electronic device 300 or a wearing environment (e.g., pressure detection in a worn state). According to an embodiment, the position or size of the display 420 and/or the pressure sensor 421 may differ from those of the illustrated embodiment depending on specifications of an electronic device (e.g., the wearable electronic device 300 of FIG. 4) to be actually manufactured. For example, the display 420 may be disposed in an area indicated by "E1" of FIG. 5 or may have a shape extending to the area indicated by "E1." In an embodiment, the display 420 may be disposed in an area indicated by "E2" of FIG. 5.

According to an embodiment, the pressure sensor 421 may include a first electrode pad 421a, a dielectric layer 421b, and/or a second electrode pad 421c. For example, the dielectric layer 421b may be provided on one surface of the first electrode pad 421a, and the second electrode pad 421c may be provided on one surface of the dielectric layer 421b so as to be disposed to face the first electrode pad 421a with the dielectric layer 421b interposed therebetween. According to an embodiment, the first electrode pad 421a and the second electrode pad 421c may be made of a metal material such as copper, and a processor (e.g., the processor 120 of FIG. 1) may detect a pressure (e.g., a wearing state of the wearable electronic device 300) based on capacitance between the first electrode pad 421a and the second electrode pad 421c. According to an embodiment, when the pressure sensor 421 is disposed to overlap the flexible display 420 and the flexible display 420 includes a metal sheet, the illustrated first electrode pad 421a may be omitted, and a metal sheet of the flexible display 420 may function as the first electrode pad 421a of the pressure sensor 421.

According to an embodiment, the valley-shaped portions 459, for example, implementing the first fastening structure 259 of FIG. 3, may be provided on an inner surface of the first wearing member 450 (e.g., the surface oriented in the -Z direction). In an embodiment, the valley-shaped portions 459 may be arranged along the length direction (e.g., the Y-axis direction). For example, the valley-shaped portions 459 may be understood as extending in a direction intersecting the length direction and being arranged along the length direction. In an embodiment, the "direction intersecting the length direction" may indicate that at least one valley-shaped portion 459 is a groove shape extending in the width direction of the wearable electronic device 300 or the X-axis direction.

In an embodiment, as will be described with reference to FIG. 8, permanent magnets (e.g., the permanent magnets 411 of FIG. 8) may be embedded in the first wearing member 450, and/or at least some of the permanent magnets 411 may be disposed inside the first wearing member 450 in an area in which the valley-shaped portions 459 are provided. In an embodiment, by a magnetic force provided by at least one permanent magnet 411, the first wearing member 450 and the second wearing member 460 may be coupled in a state of at least partially facing each other, and the mountain-shaped portions 469 of FIG. 7 may be received in one of the valley-shaped portions 459 or may be engaged with one of the valley-shaped portions 459.

According to an embodiment, as the mountain-shaped portions 469 are engaged with one of the valley-shaped portions 459, a current wearing state may be stably maintained. Here, the "current wearing state" may refer to a first wearing state in a normal mode, in which substantially no pressure is applied to a user's body (e.g., a wrist or skin), and may also refer to a second wearing state in an exercise mode, in which a greater pressure is applied to the user's body than in the normal mode in order to detect biometric signals such as an amount of exercise. For example, in the exercise mode, the wearable electronic device 300 may bring the biometric sensor module 211 of FIG. 3 into close contact with the user's body. In an embodiment, after termination of the exercise mode, the "current wearing state" may refer to a third wearing state in which a smaller pressure than that in the normal mode is applied to the user's body, or in which the wearable electronic device is sufficiently spaced apart from the user's body within a range in which the wearable electronic device does not separate from the user's body.

FIG. 7 is an enlarged perspective view illustrating a portion of a second wearing member 460 (e.g., the wearing member indicated by reference numeral "260" of FIG. 2 or FIG. 3) of a wearable electronic device (e.g., the wearable electronic device 300 of FIGS. 2 to 4) according to an embodiment of the disclosure.

Referring to FIG. 7, the second wearing member 460 may be fastened to the other side of a housing (e.g., the housing 210 of FIG. 2) by a fastening component (e.g., the locking member 261 of FIG. 3). In an embodiment, the housing 210 and the second wearing member 460 may be understood as being aligned along the length direction of the wearable electronic device 300 (e.g., the Y-axis direction, the first direction and/or the second direction of FIG. 10). For example, the first wearing member 450 may be fastened to one side of the housing 210, and the second wearing member 460 may be fastened to the other side of the housing 210, such that the first wearing member 450 and the second wearing member 460 are aligned along the length direction of the wearable electronic device 300 (e.g., the Y-axis direction, the first direction and/or the second direction of FIG. 10).

According to an embodiment, the second wearing member 460 may include mountain-shaped portions 469 that function as the second fastening structure 269 of FIG. 3. In an embodiment, the mountain-shaped portions 469 may be provided on an outer surface of the second wearing member 460 (e.g., the surface oriented in the +Z direction). For example, the first wearing member 460 may be coupled so as to substantially wrap a portion of the outer surface of the second wearing member 460, and when the first wearing member 450 and the second wearing member 460 are coupled to each other, the mountain-shaped portions 469 may be accommodated in one of the valley-shaped portions 459 or may be engaged with one of the valley-shaped portions 459. In an embodiment, the mountain-shaped portions 469 may be arranged along the length direction (e.g., the Y-axis direction, or the first direction D1 and/or the second direction D2 of FIG. 10). For example, the mountain-shaped portions 469 may be understood as extending in a direction crossing the length direction and being arranged along the length direction. In an embodiment, the "direction crossing the length direction" may indicate that at least one mountain-shaped portion 469 has a protrusion shape extending in the width direction of the wearable electronic device 300 or the X-axis direction.

According to an embodiment, as will be described with reference to FIG. 8, the second wearing member 460 may have metal pieces 419 and/or at least one electromagnet 413 embedded therein, and/or at least one electromagnet 413 may be disposed inside the second wearing member 460 in the area in which the mountain-shaped portions 469 are arranged. In an embodiment, at least one of the metal pieces 419 may generate an attractive force with at least one permanent magnet 411 embedded in the first wearing member 450. For example, the first wearing member 450 and the second wearing member 460 may be coupled to face each other by magnetic force.

According to an embodiment, a first section indicated by "E1" in FIG. 7 may illustrate a section in which the metal pieces 419 and/or the at least one electromagnet 413 are disposed. For example, when the first wearing member 450 is disposed adjacent to the first section E1, the first wearing member 450 and the second wearing member 460 may be partially coupled to face each other by an attractive force generated between the permanent magnets 411 of the first wearing member 450 and the metal pieces 419 (and/or the at least one electromagnet 413) of the second wearing member 460. In an embodiment, a second section indicated by "E2" in FIG. 7 may exemplify a section in which the first wearing member 450 is disposed to face the second wearing member 460 and is maintained in a state of being substantially in contact with the second wearing member 460 by the guide member 255. In an embodiment, one or more metal pieces 419 may be further disposed in the second section E2 (e.g., see FIG. 8, FIG. 10, or FIG. 11) to generate an attractive force that brings the first wearing member 450 into contact with the second wearing member 460.

Hereinafter, with reference to FIGS. 8 to 13, arrangements of permanent magnets 411, at least one electromagnet 413, metal pieces 419, and/or at least one geomagnetic sensor 415, and wearing states of the wearable electronic device 300 resulting therefrom will be described.

FIG. 8 is a view illustrating a first wearing state of a wearable electronic device 500 according to an embodiment of the disclosure (e.g., the wearable electronic devices 200 or 300 of FIGS. 2 to 4). FIG. 9 is a view illustrating the first wearing state of the wearable electronic device 500 according to an embodiment of the disclosure. FIG. 8 may illustrate, for example, a cross-sectional view of the wearing members 450 and 460 taken along line A-A of FIGS. 6 and 7 in a state in which the wearing members 450 and 460 are partially coupled to face each other.

Referring to FIGS. 8 and 9, the wearable electronic device 500 may include permanent magnets 411 disposed on a wearing member 520 (e.g., the first wearing member 450), at least one electromagnet 413 disposed on the wearing member 520 (e.g., the second wearing member 460), and/or metal pieces 419 disposed on the second wearing member 460. In an embodiment, the wearing member 520 may be detachably provided to a main body or a housing 510 and may be configured to allow the wearable electronic device 500 and/or the housing 510 to be worn on a user's body.

According to an embodiment, the expression "including at least one electromagnet 413" may indicate that at least one pair of electromagnets 413 is disposed on the second wearing member 460. In an embodiment, as an electrical signal is applied to the at least one electromagnet 413, the electromagnet may generate an attractive force or a repulsive force with an adjacent permanent magnet 411. The attractive force or the repulsive force between at least one electromagnet 413 and at least one permanent magnet 411 may act as a driving force for moving the first wearing member 450 and the second wearing member 460 relative to each other. For example, the wearable electronic device 500 or the processor 120 of FIG. 1 may determine whether the wearable electronic device is in a normal mode in which an exercise-related function is not executed, an exercise mode in which the exercise-related function is executed, or a state in which execution of the exercise-related function is terminated, and may adjust a degree of contact between the user's body B and the wearable electronic device 500.

According to an embodiment, the permanent magnets 411 may be disposed on the first wearing member 460 such that first polarities (e.g., S poles) and second polarities (e.g., N poles) are alternately arranged. In an embodiment, the permanent magnets 411 may be embedded in the first wearing member 450 substantially in an area in which the valley-shaped portions 459 are provided. In an embodiments described below, at least one permanent magnet 411 having the first polarity may be referred to as a first permanent magnet 411a, and at least one permanent magnet 411 having the second polarity may be referred to as a second permanent magnet 411b. In an embodiment, the metal pieces 419 may be disposed in the first section of the second wearing member 460 (e.g., the first section E1 of FIG. 7). In an embodiment, even if it is mentioned that the metal pieces 419 are generally disposed in the first section E1 of the second wearing member 460, one or more embodiments of the disclosure are not limited thereto, and at least one metal piece 419 may be additionally disposed in at least a portion of the second section (e.g., the second section E2 of FIG. 7).

According to an embodiment, when a portion in which at least one permanent magnet 411 is disposed and a portion in which at least one metal piece 419 is disposed are arranged adjacent to each other, an attractive force may be generated between the at least one permanent magnet 411 and the at least one metal piece 419, so that the first wearing member 450 and the second wearing member 460 may be at least partially coupled to face each other. When the first wearing member 450 and the second wearing member 460 are coupled to face each other, the housing 210, the first wearing member 450, and/or the second wearing member 460 may be aligned so as to substantially form a closed curve. FIGS. 8 and 9 illustrate, for example, a wearing state of the wearable electronic device 500 in a normal mode, in which a first gap G1 having a predetermined size may be provided between the wearable electronic device 500 and a user's body B (e.g., a wrist). The first gap may, for example, illustrate a gap in which the wearable electronic device 500 does not substantially press the user's body while being prevented from being separated from the user's body B.

According to an embodiment, at least one pair of electromagnets 413 may be disposed in the second wearing member 460 in an area in which the mountain-shaped portions 469 are arranged. In an embodiment, the at least one pair of electromagnets 413 may be understood as being disposed to overlap or to be in substantial contact with one of the metal pieces 419. When the wearable electronic device 500 includes a plurality of electromagnets 413, the electromagnets 413 may be understood as being arranged along the length direction of the wearable electronic device 500 (e.g., the electronic device 200 or 300 of FIGS. 2 to 4) (e.g., the Y-axis direction, or the first direction and/or the second direction of FIG. 10).

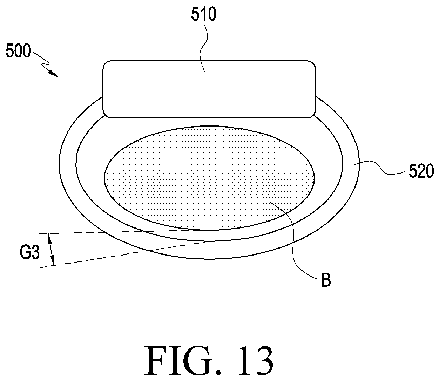

According to an embodiment, when an electrical signal is applied to the at least one pair of electromagnets 413, the electromagnets and at least one permanent magnet 411 adjacent thereto may generate an attractive force or a repulsive force. According to an embodiment, as will be described with reference to FIG. 10, in a current state (e.g., the state exemplified in FIG. 8), when a first electromagnet 413a among one or more electromagnets 413 is magnetized to an S pole and no electrical signal is applied to a second electromagnet 413b, the second wearing member 460 may move in the -Y direction, and the wearable electronic device 500 may come into close contact with a user's body B. In an embodiment, in the state exemplified in FIG. 8, when the second electromagnet 413b is magnetized to an S pole and no electrical signal is applied to the first electromagnet 413a, the second wearing member 460 may move in the +Y direction, and a gap (e.g., a third gap G3 of FIG. 13) larger than the first gap G1 may be provided between the wearable electronic device 500 and the user's body B. For example, when it is assumed that FIG. 8 exemplifies a normal mode, by controlling the first electromagnet 413a of FIG. 8 among at least one pair of electromagnets 413 to have an S-pole polarity, the second wearing member 460 may move on the first wearing member 450 in the first direction (e.g., the -Y direction) (or the first direction D1 of FIG. 10), and the wearable electronic device 500 may be changed from a wearing state of the normal mode to a wearing state of an exercise mode. In an embodiment, when it is assumed that FIG. 8 exemplifies an exercise mode, by controlling a second electromagnet 413b of FIG. 8 among the at least one pair of electromagnets 413 to have an S-pole polarity, the second wearing member 460 may move on the first wearing member 450 in the second direction (e.g., the +Y direction) (or the second direction D2 of FIG. 10), and the wearable electronic device 500 may be changed from the wearing state of the exercise mode to the wearing state of the normal mode.

According to an embodiment, the wearable electronic device 500 (e.g., the wearable electronic device 200 or 300 of FIGS. 2 to 4) may further include at least one geomagnetic sensor 415. The at least one geomagnetic sensor 415 may, for example, detect a polarity of at least one permanent magnet 411 adjacent to the at least one pair of electromagnets 413. In an embodiment, the at least one geomagnetic sensor 415 may be disposed adjacent to, to overlap, and/or to come into substantial contact with at least one electromagnet 413. Accordingly, the at least one geomagnetic sensor 415 may easily detect the polarity of the permanent magnet 411 adjacent to the at least one electromagnet 413. For example, the wearable electronic device 500 and/or the processor 120 of FIG. 1 may adjust an operation mode or a wearing state of the wearable electronic device 500 by applying an electrical signal to the at least one electromagnet 413 based on the polarity of the permanent magnet detected by the at least one geomagnetic sensor 415.

FIG. 10 is a view illustrating an operation of changing a wearing state of a wearable electronic device (e.g., the wearable electronic device 200 or 300 of FIGS. 2 to 4) according to an embodiment of the disclosure.

Referring to FIGS. 8 and 10, when it is desired to allow the wearable electronic device to come into closer contact with a user's body B, the wearable electronic device 300 (e.g., the wearable electronic device 500 of FIG. 9) or the processor 120 of FIG. 1 may magnetize the first electromagnet 413a to a second polarity, for example, an S pole. For example, the wearable electronic device 300 and/or the processor 120 of FIG. 1 may detect a polarity of at least one adjacent or surrounding permanent magnet 411 by using a geomagnetic sensor 415, and may determine a movement direction of the first wearing member 450 and/or the second wearing member 460. When the movement direction is determined, for example, when it is determined to move the second wearing member 460 in the first direction D1 (or the -Y direction), the wearable electronic device 300 or the processor 120 of FIG. 1 may magnetize the first electromagnet 413a to an S pole, such that the wearable electronic device 300 may come into closer contact with the user's body B.

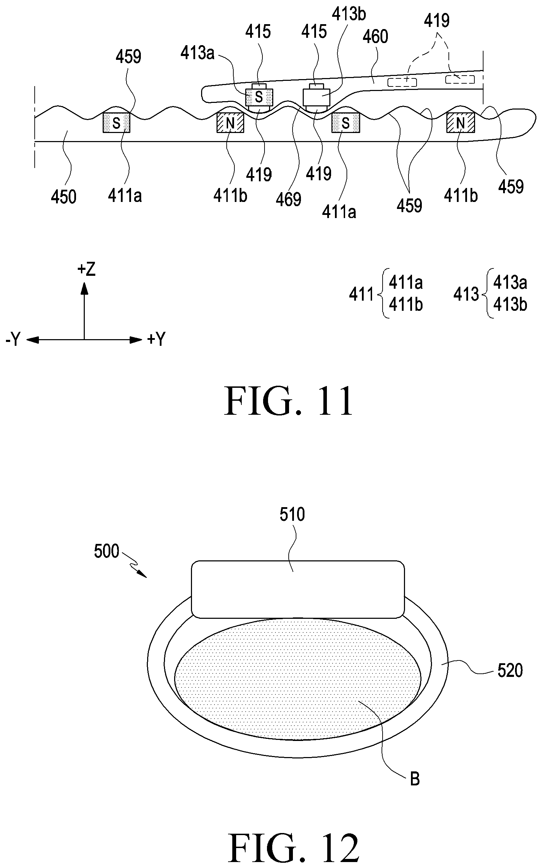

FIG. 11 is a view illustrating a second wearing state of the wearable electronic device 500 according to an embodiment of the disclosure. FIG. 12 is a view illustrating the second wearing state of the wearable electronic device 500 according to an embodiment of the disclosure.

FIGS. 11 and 12 may illustrate a state in which the wearable electronic device 500 (e.g., the wearable electronic device 200 or 300 of FIGS. 2 to 4) is in closer contact with a user's body B than that illustrated in FIGS. 8 to 10. For example, as the wearable electronic device 500 comes into closer contact with the user's body B, a gap smaller than the first gap G1 of FIG. 9 may be formed between the wearable electronic device 500 and the user's body B. In an embodiment, through the operation of FIG. 10 or FIG. 11, the wearable electronic device 500 may be substantially in close contact with the user's body, such that no gap may be present between the wearable electronic device 500 and the user's body B at the position where the first gap G1 of FIG. 9 is illustrated. In an embodiment, after the first wearing member 450 and the second wearing member 460 have moved relative to each other to a position configured in an exercise mode in which an exercise-related function is executed, an electrical signal applied to at least one pair of electromagnets 413 (e.g., the first electromagnet 413a) may be cut off or released.

According to an embodiment, in the state illustrated in FIG. 8, when an electrical signal is applied to the second electromagnet 413b such that the second electromagnet is magnetized to an S pole, and no electrical signal is applied to the first electromagnet 413a, the second wearing member 460 may move relative to the first wearing member 450 in the second direction (e.g., the +Y direction or the second direction D2 of FIG. 10). This may be understood as an operation in which the wearable electronic device 500 is worn on the user's body B while a larger gap (e.g., the third gap G3 of FIG. 13) is formed between the wearable electronic device 500 and the user's body B. This will be described in more detail with reference to FIG. 13.

FIG. 13 is a view illustrating a third wearing state of the wearable electronic device 500 (e.g., the wearable electronic device 200 or 300 of FIGS. 2 to 4) according to an embodiment of the disclosure.

Referring to FIG. 13, the wearable electronic device 500 or the processor 120 of FIG. 1 may be worn on a user's body B with a third gap G3 that is larger than the first gap G1 in a normal mode. For example, in consideration of bodily secretions generated in an exercise mode, the wearable electronic device 500 or the processor 120 of FIG. 1 may provide a sufficient gap (e.g., the third gap G3) between the user's body B and the wearable electronic device 500, thereby providing an environment in which the bodily secretions may be easily dispersed or removed.

According to an embodiment, in the state illustrated in FIG. 11, which corresponds to an exercise mode, the wearable electronic device 500 or the processor 120 of FIG. 1 may magnetize the first electromagnet 413a to an N pole and/or magnetize the second electromagnet 413b to an N pole, such that the second wearing member 460 may move relative to the first wearing member 450 in the +Y direction. For example, the wearing state of the wearable electronic device 500 may be changed from the exercise mode illustrated in FIG. 11, in which an exercise-related function is executed, to the normal mode illustrated in FIG. 8, in which the exercise-related function is not executed. According to an embodiment, when a wearing state is changed from the normal mode illustrated in FIG. 8 to the wearing state illustrated in FIG. 13, the wearable electronic device 500 or the processor 120 of FIG. 1 may magnetize the second electromagnet 413b to an N pole. In an embodiment, when the wearing state is to be changed from the normal mode of FIG. 8 to the wearing state of FIG. 13, no electrical signal may be applied to the first electromagnet 413a. For example, due to a repulsive force generated between the second electromagnet 413b and the first permanent magnet 411a adjacent thereto, or an attractive force generated between the second electromagnet 413b and the second permanent magnet 411b adjacent thereto, the second wearing member 460 may move relative to the first wearing member 450 in the +Y direction, and the wearable electronic device 500 may be changed to the wearing state of FIG. 13.

According to an embodiment, whether the wearing state has been changed to a target wearing state may be determined based on a pressure detected by a pressure sensor (e.g., the pressure sensor 421 of FIG. 5). For example, as the wearable electronic device 500 comes into closer contact with the user's body B, the pressure detected by the pressure sensor 421 may increase. In an embodiment, during an operation in which the wearing state is changed, the wearable electronic device 500 or the processor 120 of FIG. 1 may detect a change in pressure by using the pressure sensor 421.

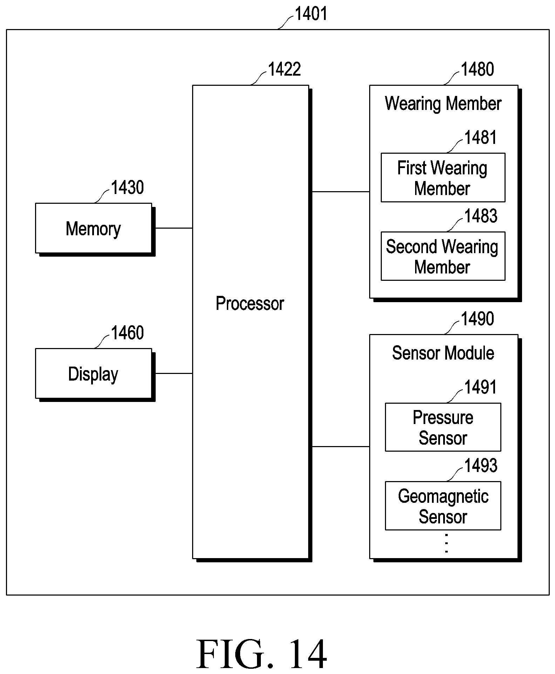

FIG. 14 is a block diagram of a wearable electronic device according to an embodiment of the disclosure.

Referring to FIG. 14, a wearable electronic device 1401 may include a processor 1422, memory 1430, a display 1460, a wearing member 1480 including a first wearing member 1481 and a second wearing member 1483, and a sensor module 1490 including a pressure sensor 1491 and a geomagnetic sensor 1493.

The wearable electronic device 1401 according to an embodiment may be a wearable electronic device that is wearable on a part of a user's body, and may represent the wearable electronic devices 200, 300, and 500 of FIGS. 9, 12, and 13.

According to an embodiment, the processor 1422 may be implemented to be substantially the same as or similar to the processor 120 of FIG. 1.

According to an embodiment, when the processor 1422 identifies execution of an exercise-related function in a first wearing state, the processor 1422 may enter a second wearing state in which a second pressure higher than a first pressure detected in the first wearing state may be detected.

According to an embodiment, when the processor 1422 detects that the wearable electronic device 1401 is worn on a part of a user's body (e.g., a user's wrist), the processor 1422 may move the second wearing member 1483 on the first wearing member 1481 to a position for detecting the first pressure. For example, the processor 1422 may detect that the electronic device 1401 is worn on a part of the user's body (e.g., a user's wrist) by using at least one electrode attached to a rear surface of the electronic device 1401 that is in contact with the part of the user's body (e.g., the user's wrist).

According to an embodiment, the processor 1422 may identify, based on first detection information received from the geomagnetic sensor 1493, that the second wearing member 1483 is moving on the first wearing member 1481 in the first direction (e.g., D1 of FIG. 10) to move to a position for detecting the first pressure.

According to an embodiment, while the second wearing member 1483 is moving in the first direction on the first wearing member 1481 to move to the position for detecting the first pressure, the processor 1422 may detect the first pressure based on second detection information received from the pressure sensor 1491.

According to an embodiment, when the processor 1422 receives the second detection information from the pressure sensor 1491, the processor 1422 may identify a pressure value from the second detection information, and when the pressure value falls within a first predetermined range for the first pressure, the processor 1422 may identify a wearing state of the electronic device as the first wearing state (e.g., the first wearing state of FIG. 9).

According to an embodiment, the first wearing state may indicate a wearing state in which various functions executable in the wearable electronic device in a normal mode in which an exercise-related function is not executed, may be executed (e.g., a call transmission/reception function or a message transmission/reception function).

According to an embodiment, when the processor 1422 identifies execution of an exercise-related function in the first wearing state, the processor 1422 may move the second wearing member 1483 on the first wearing member 1481 to a position for detecting a second pressure higher than the first pressure.

According to an embodiment, the processor 1422 may identify, based on first detection information received from the geomagnetic sensor 1493, that the second wearing member 1483 is moving on the first wearing member 1481 in the first direction (e.g., D1 of FIG. 10) to move to a position for detecting the second pressure higher than the first pressure.

According to an embodiment, the first direction for moving to the position for detecting the second pressure may be the same as the first direction for moving to the position for detecting the first pressure.

According to an embodiment, while the second wearing member 1483 is moving on the first wearing member 1481 to the position for detecting the second pressure, the processor 1422 may identify the second pressure based on second detection information received from the pressure sensor 1491.

According to an embodiment, when the second detection information is received from the pressure sensor 1491, the processor 1422 may identify a pressure value from the second detection information, and when the pressure value falls within a second predetermined range for the second pressure, the processor 1422 may identify a wearing state of the electronic device as the second wearing state (e.g., the second wearing state of FIG. 12).

According to an embodiment, the second wearing state may indicate a wearing state in which, in an exercise mode for executing an exercise recording function in which an exercise-related function is executed or in a biometric information measurement mode for executing a health measurement function, the electronic device 1401 comes into tighter contact with a part of a user's body (e.g., a user's wrist) than in the first wearing state, thereby increasing accuracy of measurement performed by at least one sensor included in the electronic device 1401 for executing the exercise-related function.

According to an embodiment, the at least one sensor for executing the exercise-related function included in the sensor module 1490 may include at least one sensor for a health measurement function (e.g., an ECG sensor, a BIA sensor, and an HRM sensor) and at least one sensor for an exercise recording function (e.g., an acceleration sensor and a gyro sensor).

According to an embodiment, when termination of execution of the exercise-related function is identified in the second wearing state, the processor 1422 may enter a third wearing state in which a third pressure lower than the first pressure detected in the first wearing state may be detected.

According to an embodiment, when termination of execution of the exercise-related function is identified in the second wearing state, the processor 1422 may move the second wearing member 1483 on the first wearing member 1481 to a position for detecting the third pressure lower than the first pressure.

According to an embodiment, the processor 1422 may identify, based on first detection information received from the geomagnetic sensor 1493, that the second wearing member 1483 is moving on the first wearing member 1481 in the second direction (e.g., D2 of FIG. 10) to move to the position for detecting the third pressure lower than the first pressure.

According to an embodiment, the second direction for moving to the position for detecting the third pressure may be a direction opposite to the first direction for moving to the position for detecting the first pressure and/or the first direction for moving to the position for detecting the second pressure.

According to an embodiment, while the second wearing member 1483 is moving on the first wearing member 1481 to the position for detecting the third pressure, the processor 1422 may identify the third pressure based on the second detection information received from the pressure sensor 1491.

According to an embodiment, when the second detection information is received from the pressure sensor 1491, the processor 1422 may identify a pressure value from the second detection information, and when the pressure value falls within a third predetermined range for the third pressure, the processor 1422 may identify a wearing state of the electronic device as the third wearing state (e.g., the third wearing state of FIG. 13).

According to an embodiment, the third wearing state may indicate a wearing state that is looser than the first wearing state when execution of an exercise-related function is terminated, so as to prevent occurrence of skin irritation on a part of a user's body (e.g., a user's wrist) that has been tightly in close contact with the electronic device at the second pressure while the exercise-related function is being executed.

According to an embodiment, when the processor 1422 identifies that a predetermined time has elapsed in the third wearing state, the processor 1422 may enter the first wearing state.

According to an embodiment, upon identifying that the predetermined time has elapsed in the third wearing state, the processor 1422 may enter the first wearing state in which the second wearing member 1483 is moved on the first wearing member 1481 to a position for detecting the first pressure.

According to an embodiment, depending on a type of an exercise-related function executed in the second wearing state, when termination of execution of the exercise-related function is identified, the processor 1422 may enter the first wearing state after identifying that a predetermined time has elapsed since entering the third wearing state from the second wearing state, or may enter the first wearing state directly from the second wearing state.

According to an embodiment, when the exercise-related function in the second wearing state is an exercise recording function, the processor 1422 may enter the third wearing state upon identifying termination of execution of the exercise recording function, and may enter the first wearing state after identifying that a predetermined time has elapsed.

According to an embodiment, when the exercise-related function in the second wearing state is a health measurement function, the processor 1422 may enter the first wearing state (e.g., the first wearing state of FIG. 9) upon identifying termination of execution of the health measurement function.

According to an embodiment, the memory 1430 may be implemented to be substantially the same as or similar to the memory 130 of FIG. 1.

According to an embodiment, the memory 1430 may store data or instructions of the wearable electronic device 1401. For example, instructions stored in the memory 1430 may be configured to cause the processor 1422 to perform specific operations.

According to an embodiment, the memory 1430 may include a table related to pressure data for the first wearing state (e.g., the first predetermined range for the first pressure), pressure data for the second wearing state (e.g., the second predetermined range for the second pressure), and pressure data for the third wearing state (e.g., the third predetermined range for the third pressure).

According to an embodiment, the display 1460 may be implemented to be substantially the same as or similar to the display 160 of FIG. 1 and/or the flexible display 420 of FIG. 5.

According to an embodiment, the wearing member 1480 may be implemented to be substantially the same as or similar to the wearing member 520 of FIGS. 9, 12, and 13.

According to an embodiment, the first wearing member 1481 of the wearing member 1480 may be implemented to be substantially the same as or similar to the first wearing member 150 of FIGS. 2 and 3, the first wearing member 295 of FIG. 4, and the first wearing member 450 of FIGS. 5, 6, 10, and 11.

According to an embodiment, the second wearing member 1483 of the wearing member 1480 may be implemented to be substantially the same as or similar to the second wearing member 160 of FIGS. 2 and 3, the second wearing member 297 of FIG. 4, and the second wearing member 460 of FIGS. 7, 8, 10, and 11.

According to an embodiment, the sensor module 1490 may be implemented to be substantially the same as or similar to the sensor module 176 of FIG. 1.

According to an embodiment, the pressure sensor 1491 of the sensor module 1490 may be implemented to be substantially the same as or similar to the pressure sensor 421 of FIG. 5.

According to an embodiment, the pressure sensor 1491 may detect pressure (e.g., the first pressure, the second pressure, or the third pressure) while the second wearing member 1483 moves on the first wearing member 1481 in the first direction (e.g., D1 of FIG. 10) or the second direction (e.g., D2 of FIG. 10).

According to an embodiment, the geomagnetic sensor 1493 of the sensor module 1490 may be implemented to be substantially the same as or similar to the geomagnetic sensor 415 of FIGS. 8, 10, and 11.

According to an embodiment, the geomagnetic sensor 1493 may detect a direction in which the second wearing member 1483 moves on the first wearing member 1481.

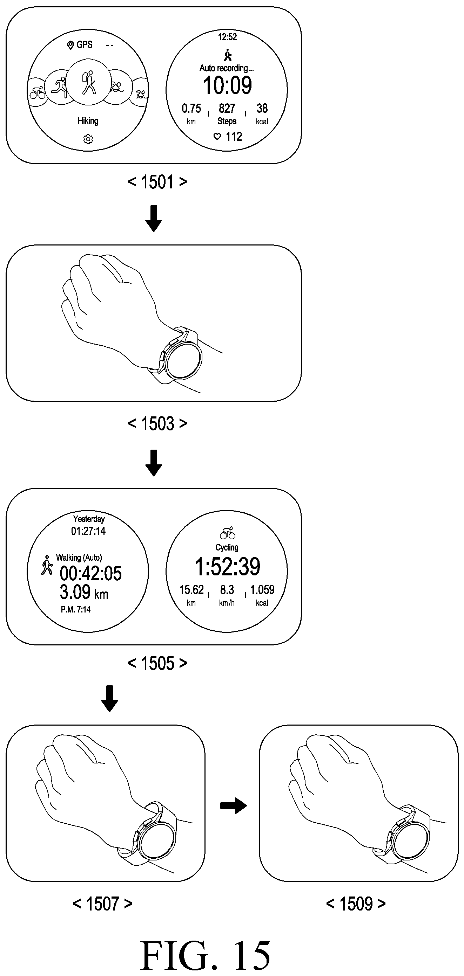

FIG. 15 is a view illustrating an operation of entering a wearing state according to an exercise-related function in a wearable electronic device according to an embodiment of the disclosure.

Referring to FIG. 15, as illustrated in screen 1501, according to an embodiment, in the first wearing state in which the electronic device is worn on a part of a user's body (e.g., a user's wrist) and is capable of detecting the first pressure, an electronic device(e.g., the electronic device 101 of FIG. 1 and/or the electronic device 201 of FIG. 2) may identify execution of an exercise recording function among exercise-related functions. According to an embodiment, the electronic device may identify execution of the exercise recording function based on a user's selection. According to an embodiment, the electronic device may identify execution of an exercise recording function when a user wearing the electronic device on a part of the user's body performs an action detectable as exercise for a predetermined time.

As illustrated in screen 1503, according to an embodiment, upon identifying execution of the exercise recording function among exercise-related functions, the electronic device (e.g., the electronic device 101 of FIG. 1 and/or the electronic device 201 of FIG. 2) may enter the second wearing state capable of detecting the second pressure higher than the first pressure from the first wearing state capable of detecting the first pressure.

As illustrated in screen 1505, according to an embodiment, the electronic device (e.g., the electronic device 101 of FIG. 1 and/or the electronic device 201 of FIG. 2) may identify termination of execution of the exercise recording function among the exercise-related functions. According to an embodiment, the electronic device may identify termination of execution of the exercise recording function based on a user's selection. According to an embodiment, the electronic device may identify termination of execution of the exercise recording function when a user wearing the electronic device on a part of the user's body no longer performs an action detectable as exercise for a predetermined time. According to an embodiment, upon identifying termination of execution of the exercise recording function among the exercise-related functions, the electronic device may terminate exercise recording and display recorded exercise information on a display of the electronic device (e.g., the display 1060 of FIG. 10).

As illustrated in screen 1507, according to an embodiment, upon identifying termination of execution of the exercise recording function among the exercise-related functions in the second wearing state capable of detecting the second pressure, the electronic device (e.g., the electronic device 101 of FIG. 1 and/or the electronic device 201 of FIG. 2), may enter the third wearing state capable of detecting the third pressure lower than the first pressure.

As illustrated in screen 1509, according to an embodiment, upon identifying that a designated time has elapsed in the third wearing state so as to prevent occurrence of skin irritation of a user due to tight contact between the electronic device and a part of the user's body (e.g., the user's wrist) during the second wearing state, the electronic device (e.g., the electronic device 101 of FIG. 1 and/or the electronic device 201 of FIG. 2) may enter the first wearing state capable of detecting the first pressure.

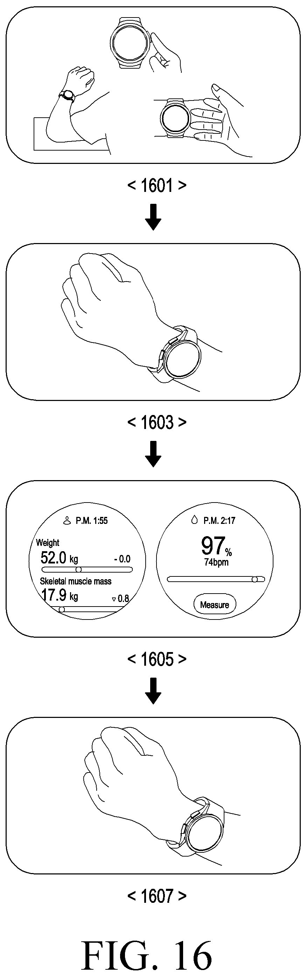

FIG. 16 is a view illustrating an operation of entering a wearing state according to an exercise-related function in a wearable electronic device according to an embodiment of the disclosure.

Referring to FIG. 16, as illustrated in screen 1601, an electronic device according to an embodiment (e.g., the electronic device 101 of FIG. 1 and/or the electronic device 201 of FIG. 2), in the first wearing state in which the electronic device is worn on a part of a user's body (e.g., a user's wrist) and is capable of detecting the first pressure, may identify execution of a health measurement function among exercise-related functions. According to an embodiment, the electronic device may identify execution of the health measurement recording function based on a user's selection. According to an embodiment, after the health recording function is selected on the electronic device, the electronic device may execute the health measurement recording function through a user's touch on at least one sensor for health recording in the electronic device.

As illustrated in screen 1603, according to an embodiment, upon identifying execution of the health recording function among the exercise-related functions, the electronic device (e.g., the electronic device 101 of FIG. 1 and/or the electronic device 201 of FIG. 2) may enter the second wearing state capable of detecting the second pressure higher than the first pressure from the first wearing state capable of detecting the first pressure.

As illustrated in screen 1605, according to an embodiment, the electronic device (e.g., the electronic device 101 of FIG. 1 and/or the electronic device 201 of FIG. 2) may identify termination of execution of the health recording function among the exercise-related functions. According to an embodiment, the electronic device may identify termination of execution of the health recording function based on a user's selection on the electronic device. According to an embodiment, upon identifying termination of execution of the health recording function among the exercise-related functions, the electronic device may terminate health recording and may display recorded health information on a display thereof (e.g., the display 1060 of FIG. 10).

As illustrated in screen 1507, according to an embodiment, upon identifying termination of execution of the health recording function among the exercise-related functions in the second wearing state capable of detecting the second pressure, the electronic device (e.g., the electronic device 101 of FIG. 1 and/or the electronic device 201 of FIG. 2) may enter the first wearing state capable of detecting the first pressure.

FIG. 17 is a flowchart illustrating operations of entering wearing states according to an exercise-related function in a wearable electronic device according to an embodiment of the disclosure. The operations of entering wearing states according to an exercise-related function in the electronic device may include operations 1701 to 17103. In the following embodiments, individual operations may be performed sequentially, but are not necessarily performed sequentially. For example, an order of the operations may be changed, at least two operations may be performed in parallel, or another operation may be added.

In operation 1701, an electronic device (e.g., the electronic device 101 of FIG. 1, the electronic devices of FIGS. 9, 12, and 13, and/or the electronic device 1001 of FIG. 14) may detect wearing of the electronic device on a part of a user's body.

According to an embodiment, the electronic device may detect wearing of the electronic device on a part of the user's body (e.g., the user's wrist) by using at least one electrode attached to a rear surface of the electronic device that is in contact with the part of the user's body (e.g., the user's wrist).

In operation 1703, the electronic device (e.g., the electronic device 101 of FIG. 1, the electronic devices of FIGS. 9, 12, and 13, and/or the electronic device 1001 of FIG. 14) may enter a first wearing state (e.g., the first wearing state of FIG. 9).

According to an embodiment, upon detecting the wearing of the electronic device on a part of a user's body (e.g., the user's wrist), the electronic device may move a second wearing member (e.g., the second wearing member 1083 of FIG. 10) on a first wearing member (e.g., the first wearing member 1081 of FIG. 10) to a position for detecting a first pressure.

According to an embodiment, based on first detection information received from a geomagnetic sensor (e.g., the geomagnetic sensor 1493 of FIG. 14), the electronic device may detect that the second wearing member is moving on the first wearing member in a first direction (e.g., D1 of FIG. 10) to move to the position for detecting the first pressure.

According to an embodiment, while the second wearing member is moving on the first wearing member to the position for detecting the first pressure, the electronic device may identify the first pressure based on second detection information received from a pressure sensor (e.g., the pressure sensor 1491 of FIG. 14).

According to an embodiment, upon receiving the second detection information from the pressure sensor, the electronic device may identify a pressure value from the second detection information, and when the pressure value falls within a first predetermined range for the first pressure, the electronic device may identify the wearing state of the electronic device as the first wearing state.

In operation 1705, the electronic device (e.g., the electronic device 101 of FIG. 1, the electronic devices of FIGS. 9, 12, and 13, and/or the electronic device 1001 of FIG. 14) may identify whether an exercise-related function is executed.

In operation 1705, upon failing to identify execution of the exercise-related function in the electronic device, the electronic device may maintain the first wearing state in operation 1703.

In operation 1705, upon identifying execution of the exercise-related function in the electronic device, the electronic device may enter a second wearing state (e.g., the second wearing state of FIG. 12) in operation 1707.

According to an embodiment, upon identifying execution of the exercise-related function in the first wearing state, the electronic device may move the second wearing member on the first wearing member to a position for detecting a second pressure higher than the first pressure.

According to an embodiment, based on first detection information received from a geomagnetic sensor (e.g., the geomagnetic sensor 1493 of FIG. 14), the electronic device may identify that the second wearing member is moving on the first wearing member in the first direction (e.g., D1 of FIG. 10) to move to the position for detecting the second pressure higher than the first pressure.

According to an embodiment, while the second wearing member is moving on the first wearing member in a direction for detecting the second pressure, the electronic device may identify the second pressure based on second detection information received from the pressure sensor (e.g., the geomagnetic sensor 1491 of FIG. 14).

According to an embodiment, upon receiving the second detection information from the pressure sensor, the electronic device may identify a pressure value from the second detection information, and when the pressure value falls within a second predetermined range for the second pressure, the electronic device may identify the wearing state of the electronic device as the second wearing state.

In operation 1709, the electronic device (e.g., the electronic device 101 of FIG. 1, the electronic devices of FIGS. 9, 12, and 13, and/or the electronic device 1001 of FIG. 14) may identify whether execution of an exercise-related function is terminated.

In operation 1709, upon failing to identify termination of execution of the exercise-related function, the electronic device may maintain execution of the exercise-related function in the second wearing state in operation 1707.

In operation 1709, upon identifying termination of execution of the exercise-related function, the electronic device may enter a third wearing state (e.g., the third wearing state of FIG. 13) in operation 1711.

According to an embodiment, upon identifying termination of execution of the exercise-related function in the second wearing state, the electronic device may enter the third wearing state capable of detecting a third pressure lower than the first pressure detected in the first wearing state.

According to an embodiment, upon identifying termination of execution of the exercise-related function in the second wearing state, the electronic device may move the second wearing member on the first wearing member to a position for detecting the third pressure lower than the first pressure.

According to an embodiment, based on second detection information received from a geomagnetic sensor (e.g., the geomagnetic sensor 1493 of FIG. 14), the electronic device may identify that the second wearing member is moving on the first wearing member in the second direction (e.g., D2 of FIG. 10) to move to the position for detecting the third pressure lower than the first pressure.

According to an embodiment, while the second wearing member is moving on the first wearing member to a position for detecting the third pressure, the electronic device may identify the third pressure based on second detection information received from the pressure sensor (e.g., the geomagnetic sensor 1491 of FIG. 14).

According to an embodiment, upon receiving the second detection information from the pressure sensor, the electronic device may identify a pressure value from the second detection information, and when the pressure value falls within a third predetermined range for the third pressure, the electronic device may identify the wearing state of the electronic device as the third wearing state.

In operation 1713, the electronic device (e.g., the electronic device 101 of FIG. 1, the electronic devices of FIGS. 9, 12, and 13, and/or the electronic device 1001 of FIG. 14) may identify whether a predetermined time has elapsed.

In operation 1713, upon failing to identify that the predetermined time has elapsed, the electronic device may maintain the third wearing state in operation 1711.

In operation 1713, upon identifying that the predetermined time has elapsed, the electronic device may enter the first wearing state in operation 1703.

According to an embodiment, upon identifying that the predetermined time has elapsed in the third wearing state, the electronic device may enter the first wearing state (e.g., the first wearing state of FIG. 9).

According to an embodiment, upon identifying that the predetermined time has elapsed in the third wearing state, the electronic device may enter the first wearing state in which the second wearing member is moved on the first wearing member in a direction for detecting the first pressure.

As described above, according to one or more embodiments of the disclosure, a wearable electronic device (e.g., the wearable electronic devices 200, 300, and 500 of FIGS. 2 to 4, FIG. 9, FIG. 12, and/or FIG. 13) may include at least one permanent magnet (e.g., the permanent magnets 411 of FIG. 10) and/or at least one electromagnet (e.g., the electromagnets 413 of FIG. 10), thereby easily adjusting a wearing state thereof. For example, when biometric information is to be detected while providing wearing comfort in daily life, the wearable electronic device may be brought into close contact with a user's body, thereby increasing accuracy of the detected biometric information. In an embodiment, after an exercise mode is terminated, a sufficient gap may be provided between the user's body and the wearable electronic device, thereby facilitating removal of bodily secretions. For example, contamination of the wearable electronic device due to bodily secretions or injury to the user may be suppressed.

The effects obtainable from the disclosure are not limited to those described above, and other effects not mentioned herein will be clearly understood by a person ordinarily skilled in the art to which the disclosure pertains from the description of one or more embodiments described above.

According to an embodiment of the disclosure, a wearable electronic device (e.g., the wearable electronic device 200, 300, 500, or 1401 of FIGS. 2 to 4, FIG. 9, FIG. 12, FIG. 13, and/or FIG. 14) may include a housing (e.g., the housing 210 or 510 of FIG. 2 or FIG. 9), a first wearing member (e.g., the first wearing member 450 of FIG. 10) detachably disposed on one side of the housing, and a second wearing member (e.g., the second wearing member 460 of FIG. 10) detachably disposed on the other side of the housing and aligned with the first wearing member along a first direction (e.g., the Y-axis direction of FIG. 4 or the first direction D1 of FIG. 10) with the housing interposed therebetween, the second wearing member being coupled so as to at least partially face the first wearing member, thereby forming a closed loop together with the housing and the first wearing member. The wearable electronic device may further include first magnetic bodies (e.g., the permanent magnets 411 of FIG. 10) disposed on the first wearing member such that first polarities and second polarities are alternately arranged along the first direction, and at least one pair of second magnetic bodies (e.g., the electromagnets 413 of FIG. 10) arranged on the second wearing member along the first direction. In an embodiment, the at least one pair of second magnetic bodies may be configured to generate an attractive force or a repulsive force with the first magnetic bodies in response to receiving an electrical signal.

According to an embodiment, the first magnetic bodies may include permanent magnets, and the second magnetic bodies may include electromagnets. In an embodiment, the first wearing member and the second wearing member may be configured to move relative to each other in the first direction or in a second direction (e.g., the second direction D2 of FIG. 10) opposite to the first direction by an attractive force or a repulsive force generated between the at least one pair of second magnetic bodies and the first magnetic bodies.

According to an embodiment, the wearable electronic device as described above may further include a pressure sensor (e.g., the pressure sensor 421 of FIG. 5) embedded in one of the first wearing member and the second wearing member.

According to an embodiment, the first wearing member and the second wearing member may be configured to move in the first direction or in the second direction opposite to the first direction by an attractive force or a repulsive force generated between the at least one pair of second magnetic bodies and one of the first magnetic bodies. In an embodiment, the pressure sensor may be configured to detect a pressure or a change in the pressure according to a relative movement between the first wearing member and the second wearing member.

According to an embodiment, the pressure sensor may include a first electrode pad (e.g., the first electrode pad 421a of FIG. 5), a dielectric layer (e.g., the dielectric layer 421c of FIG. 5) disposed on one surface of the first electrode pad, and a second electrode pad (e.g., the second electrode pad 421c of FIG. 5) disposed on one surface of the dielectric layer and facing the first electrode pad with the dielectric layer interposed therebetween.

According to an embodiment, the wearable electronic device as described above may further include at least one geomagnetic sensor (e.g., the geomagnetic sensor 415 of FIG. 10) disposed on the second wearing member. In an embodiment, the at least one geomagnetic sensor may be configured to detect a polarity of at least one of the first magnetic bodies that is adjacent to the at least one pair of second magnetic bodies.

According to an embodiment, the at least one geomagnetic sensor may be disposed so as to overlap one of the at least one pair of second magnetic bodies.

According to an embodiment, the wearable electronic device as described above may further include valley-shaped portions (e.g., the valley-shaped portions 459 of FIG. 10) formed on one of the first wearing member and the second wearing member, the valley-shaped portions extending in a direction crossing the first direction and being arranged along the first direction, and mountain-shaped portions (e.g., the mountain-shaped portions 469 of FIG. 10) formed on the other one of the first wearing member and the second wearing member, the mountain-shaped portions extending in a direction crossing the first direction and being arranged along the first direction. According to an embodiment, when the second wearing member is coupled with the first wearing member to at least partially face the first wearing member, at least one of the mountain-shaped portions may be configured to be accommodated in one of the valley-shaped portions or to be engaged with one of the valley-shaped portions.

According to an embodiment, the wearable electronic device as described above may further include a flexible display (e.g., the flexible display 420 of FIG. 5) disposed on at least one of the first wearing member and the second wearing member.

According to an embodiment, the wearable electronic device as described above may further include a processor (e.g., the processor 120 or 1422 of FIG. 1 and/or FIG. 14) and memory (e.g., the memory 130 or 1430 of FIG. 1 and/or FIG. 14) configured to store instructions. According to an embodiment, the instructions, when executed by the processor, may be configured to cause the wearable electronic device to enter a second wearing state in which the second wearing member is moved on the first wearing member to a position for detecting a second pressure higher than the first pressure when execution of an exercise-related function is identified in a first wearing state in which the second wearing member is moved on the first wearing member to a position for detecting a first pressure. According to an embodiment, the instructions, when executed by the processor, may be configured to cause the wearable electronic device to enter a third wearing state in which the second wearing member is moved on the first wearing member to a position for detecting a third pressure lower than the first pressure when termination of execution of the exercise-related function is identified in the second wearing state.

According to an embodiment, the instructions, when executed by the processor, may be configured to cause the wearable electronic device to enter the first wearing state when a predetermined time has elapsed in the third wearing state.

According to an embodiment, the instructions, when executed by the processor, may be configured to cause the wearable electronic device to enter the first wearing state when it is detected that the wearable electronic device is worn on a part of a user's body.

According to an embodiment, the instructions, when executed by the processor, may be configured to cause the wearable electronic device to identify a movement direction of the second wearing member moving on the first wearing member based on first detection information received from a geomagnetic sensor.

According to an embodiment, the instructions, when executed by the processor, may be configured to cause the wearable electronic device to identify a pressure value from second detection information received from a pressure sensor. According to an embodiment, the instructions, when executed by the processor, may be configured to cause the wearable electronic device to identify a wearing state as the first wearing state when the pressure value falls within a first predetermined range for the first pressure. According to an embodiment, the instructions, when executed by the processor, may be configured to cause the wearable electronic device to identify a wearing state as the second wearing state when the pressure value falls within a second predetermined range for the second pressure. According to an embodiment, the instructions, when executed by the processor, may be configured to cause the wearable electronic device to identify a wearing state as the third wearing state when the pressure value falls within a third predetermined range for the third pressure.

According to an embodiment of the disclosure, a wearable electronic device (e.g., the wearable electronic device 200, 300, 500, or 1401 of FIGS. 2 to 4, FIG. 9, FIG. 12, FIG. 13, and/or FIG. 14) may include a housing (e.g., the housing 210 or 510 of FIG. 2 or FIG. 9), a first wearing member (e.g., the first wearing member 450 of FIG. 10) detachably disposed on one side of the housing, and a second wearing member (e.g., the second wearing member 460 of FIG. 10) detachably disposed on the other side of the housing and aligned with the first wearing member along a first direction (e.g., the Y-axis direction of FIG. 4 or the first direction D1 of FIG. 10) with the housing interposed therebetween, the second wearing member being coupled so as to at least partially face the first wearing member, thereby forming a closed loop together with the housing and the first wearing member. The wearable electronic device may further include permanent magnets (e.g., the permanent magnets 411 of FIG. 10) disposed on the first wearing member such that first polarities and second polarities are alternately arranged along the first direction, and at least one pair of electromagnets (e.g., the electromagnets 413 of FIG. 10) arranged on the second wearing member along the first direction. According to an embodiment, the at least one pair of electromagnets may be configured to generate an attractive force or a repulsive force with the permanent magnets in response to receiving an electrical signal.

According to an embodiment, the first wearing member and the second wearing member may be configured to move relative to each other in the first direction or in a second direction (e.g., the second direction D2 of FIG. 10) opposite to the first direction by the attractive force or the repulsive force generated between the at least one pair of electromagnets and the permanent magnets.

According to an embodiment, the wearable electronic device as described above may further include a pressure sensor (e.g., the pressure sensor 421 of FIG. 5) embedded in one of the first wearing member and the second wearing member.

According to an embodiment, the first wearing member and the second wearing member may be configured to move in the first direction or in the second direction opposite to the first direction by an attractive force or a repulsive force generated between the at least one pair of electromagnets and one of the permanent magnets. According to an embodiment, the pressure sensor may be configured to detect a pressure or a change in the pressure according to a relative movement between the first wearing member and the second wearing member.

According to an embodiment, the pressure sensor may include a first electrode pad (e.g., the first electrode pad 421a of FIG. 5), a dielectric layer (e.g., the dielectric layer 421c of FIG. 5) disposed on one surface of the first electrode pad, and a second electrode pad (e.g., the second electrode pad 421c of FIG. 5) disposed on one surface of the dielectric layer and facing the first electrode pad with the dielectric layer interposed therebetween.

According to an embodiment, the wearable electronic device as described above may further include at least one geomagnetic sensor (e.g., the geomagnetic sensor 415 of FIG. 10) disposed on the second wearing member. In an embodiment, the at least one geomagnetic sensor may be configured to detect a polarity of at least one of the permanent magnets that is adjacent to the at least one pair of electromagnets.

According to an embodiment, the at least one geomagnetic sensor may be disposed so as to overlap one of the at least one pair of electromagnets.

According to an embodiment, the wearable electronic device as described above may further include valley-shaped portions (e.g., the valley-shaped portions 459 of FIG. 10) formed on one of the first wearing member and the second wearing member, the valley-shaped portions extending in a direction crossing the first direction and being arranged along the first direction, and mountain-shaped portions (e.g., the mountain-shaped portions 469 of FIG. 10) formed on the other one of the first wearing member and the second wearing member, the mountain-shaped portions extending in a direction crossing the first direction and being arranged along the first direction. In an embodiment, when the second wearing member is coupled with the first wearing member to at least partially face the first wearing member, at least one of the mountain-shaped portions may be configured to be accommodated in one of the valley-shaped portions or to be engaged with one of the valley-shaped portions.

According to an embodiment, the wearable electronic device as described above may further include a flexible display (e.g., the flexible display 420 of FIG. 5) disposed on at least one of the first wearing member and the second wearing member.

According to an embodiment, the wearable electronic device as described above may further include a processor (e.g., the processor 120 or 1422 of FIG. 1 and/or FIG. 14) and memory (e.g., the memory 130 or 1430 of FIG. 1 and/or FIG. 14) configured to store instructions. According to an embodiment, the instructions, when executed by the processor, may be configured to cause the wearable electronic device to enter a second wearing state in which the second wearing member is moved on the first wearing member to a position for detecting a second pressure higher than the first pressure when execution of an exercise-related function is identified in a first wearing state in which the second wearing member is moved on the first wearing member to a position for detecting a first pressure. According to an embodiment, the instructions, when executed by the processor, may be configured to cause the wearable electronic device to enter a third wearing state in which the second wearing member is moved on the first wearing member to a position for detecting a third pressure lower than the first pressure when termination of execution of the exercise-related function is identified in the second wearing state.

According to an embodiment, the instructions, when executed by the processor, may be configured to cause the wearable electronic device to enter the first wearing state when a predetermined time has elapsed in the third wearing state.

According to an embodiment, the instructions, when executed by the processor, may be configured to cause the wearable electronic device to enter the first wearing state when it is detected that the wearable electronic device is worn on a part of a user's body.

According to an embodiment, the instructions, when executed by the processor, may be configured to cause the wearable electronic device to identify a movement direction of the second wearing member moving on the first wearing member based on first detection information received from a geomagnetic sensor.

According to an embodiment, the instructions, when executed by the processor, may be configured to cause the wearable electronic device to identify a pressure value from second detection information received from a pressure sensor. According to an embodiment, the instructions, when executed by the processor, may be configured to cause the wearable electronic device to identify a wearing state as the first wearing state when the pressure value falls within a first predetermined range for the first pressure. According to an embodiment, the instructions, when executed by the processor, may be configured to cause the wearable electronic device to identify a wearing state as the second wearing state when the pressure value falls within a second predetermined range for the second pressure. According to an embodiment, the instructions, when executed by the processor, may be configured to cause the wearable electronic device to identify a wearing state as the third wearing state when the pressure value falls within a third predetermined range for the third pressure.

According to an embodiment of the disclosure, a wearable electronic device (e.g., the wearable electronic device 200, 300, or 500 of FIGS. 2 to 4, FIG. 9, FIG. 12, and/or FIG. 13) may include a housing (e.g., the housing 210 or 510 of FIG. 2 or FIG. 9), a first wearing member (e.g., the first wearing member 450 of FIG. 10) detachably disposed on one side of the housing, and a second wearing member (e.g., the second wearing member 460 of FIG. 10) detachably disposed on the other side of the housing and aligned with the first wearing member along a first direction (e.g., the Y-axis direction of FIG. 4 or the first direction D1 of FIG. 10) with the housing interposed therebetween. The second wearing member may be configured to be coupled so as to at least partially face the first wearing member, thereby forming a closed loop together with the housing and the first wearing member. The wearable electronic device may further include permanent magnets (e.g., the permanent magnets 411 of FIG. 10) disposed on the first wearing member, and at least one pair of electromagnets (e.g., the electromagnets 413 of FIG. 10) arranged on the second wearing member along the first direction. According to an embodiment, the at least one pair of electromagnets may be configured to receive an electrical signal and generate an attractive force or a repulsive force with the permanent magnets, thereby moving the first wearing member and the second wearing member relative to each other in the first direction or in a second direction (e.g., the second direction D2 of FIG. 10) opposite to the first direction.

According to an embodiment, the wearable electronic device as described above may further include a pressure sensor (e.g., the pressure sensor 421 of FIG. 5) embedded in one of the first wearing member and the second wearing member. According to an embodiment, the first wearing member and the second wearing member may be configured to move in the first direction or in the second direction opposite to the first direction by an attractive force or a repulsive force generated between the at least one pair of electromagnets and one of the permanent magnets. According to an embodiment, the pressure sensor may be configured to detect a pressure or a change in the pressure according to a relative movement between the first wearing member and the second wearing member.

According to an embodiment, the pressure sensor may include a first electrode pad (e.g., the first electrode pad 421a of FIG. 5), a dielectric layer (e.g., the dielectric layer 421c of FIG. 5) disposed on one surface of the first electrode pad, and a second electrode pad (e.g., the second electrode pad 421c of FIG. 5) disposed on one surface of the dielectric layer and facing the first electrode pad with the dielectric layer interposed therebetween.

According to an embodiment, the wearable electronic device as described above may further include at least one geomagnetic sensor (e.g., the geomagnetic sensor 415 of FIG. 10) disposed on the second wearing member. In an embodiment, the at least one geomagnetic sensor may be configured to detect a polarity of at least one of the permanent magnets that is adjacent to the at least one pair of electromagnets.

According to an embodiment, the wearable electronic device as described above may further include valley-shaped portions (e.g., the valley-shaped portions 459 of FIG. 10) formed on one of the first wearing member and the second wearing member, the valley-shaped portions extending in a direction crossing the first direction and being arranged along the first direction, and mountain-shaped portions (e.g., the mountain-shaped portions 469 of FIG. 10) formed on the other one of the first wearing member and the second wearing member, the mountain-shaped portions extending in a direction crossing the first direction and being arranged along the first direction. In an embodiment, when the second wearing member is coupled with the first wearing member to at least partially face the first wearing member, at least one of the mountain-shaped portions may be configured to be accommodated in one of the valley-shaped portions or to be engaged with one of the valley-shaped portions.

According to an embodiment, the wearable electronic device as described above may further include a processor (e.g., the processor 120 of FIG. 1) and memory (e.g., the memory 130 of FIG. 1) configured to store instructions. According to an embodiment, the instructions, when executed by the processor, may be configured to cause the wearable electronic device to enter a second wearing state in which the second wearing member is moved on the first wearing member to a position for detecting a second pressure higher than the first pressure when execution of an exercise-related function is identified in a first wearing state in which the second wearing member is moved on the first wearing member to a position for detecting a first pressure. According to an embodiment, the instructions, when executed by the processor, may be configured to cause the wearable electronic device to enter a third wearing state in which the second wearing member is moved on the first wearing member to a position for detecting a third pressure lower than the first pressure when termination of execution of the exercise-related function is identified in the second wearing state.

Although the disclosure has been described with reference to an embodiment as an example, it is to be understood that the embodiment is intended to be exemplary and is not limiting the disclosure. It will be apparent to those skilled in the art that various changes in form and detail may be made without departing from the overall scope of the disclosure, including the appended claims and their equivalents. For example, in the above-described embodiment, a configuration in which one or more valley-shaped portions are formed on the first wearing member and one or more mountain-shaped portions are formed on the second wearing member has been illustrated. However, embodiments of the disclosure are not limited thereto, and in other embodiments not illustrated, the one or more valley-shaped portions may be formed on the second wearing member, and the one or more mountain-shaped portions may be formed on the first wearing member.

## Claims

1. A wearable electronic device (101; 200; 300; 500; 1401), comprising:
a housing (210; 510);
a first wearing member (250; 450) detachably disposed on one side of the housing;
a second wearing member (2620; 460) detachably disposed on another side of the housing and aligned with the first wearing member in a first direction (D) with the housing interposed therebetween, the second wearing member configured to be capable of forming a closed curve with the housing and the first wearing member by being coupled with the first wearing member while at least partially facing the first wearing member;
first magnetic bodies (411) arranged on the first wearing member so that first polarities and second polarities of the first magnetic bodies are alternately arranged along the first direction; and
at least one pair of second magnetic bodies (413) arranged in the second wearing member along the first direction,
wherein the at least one pair of second magnetic bodies are configured to generate attractive force or repulsive force with the first magnetic bodies based on receiving an electric signal.

2. The wearable electronic device of claim 1, wherein the first magnetic bodies include permanent magnets and the at least one pair of second magnetic bodies include electromagnets, and
wherein the first wearing member and the second wearing member are configured to move in the first direction or a second direction (D2) opposite to the first direction with respect to each other, by attractive force or repulsive force generated between the at least one pair of second magnetic bodies and the first magnetic bodies.

3. The wearable electronic device of any one of claims 1 and 2, further comprising:
a pressure sensor (421) disposed in one of the first wearing member or the second wearing member.

4. The wearable electronic device of claim 3, wherein the first wearing member and the second wearing member are configured to move in the first direction or a second direction opposite to the first direction with respect to each other, by attractive force or repulsive force generated between the at least one pair of second magnetic bodies and the first magnetic bodies, and
wherein the pressure sensor is configured to detect a pressure or a change in pressure according to a relative movement of the first wearing member and the second wearing member.

5. The wearable electronic device of any one of claims 3 and 4, wherein the pressure sensor comprises:
a first electrode pad (421a);
a dielectric layer (421b) disposed on one surface of the first electrode pad; and
a second electrode pad (421c) disposed on one surface of the dielectric layer and facing the first electrode pad with the dielectric layer interposed therebetween.

6. The wearable electronic device of any one of claims 1 to 5, further comprising:
at least one geomagnetic sensor (415) disposed on the second wearing member,
wherein the at least one geomagnetic sensor is configured to detect a polarity of at least one of the first magnetic bodies adjacent to the at least one pair of second magnetic bodies.

7. The wearable electronic device of claim 6, wherein the at least one geomagnetic sensor is disposed to overlap one of the at least one pair of second magnetic bodies.

8. The wearable electronic device of any one of claims 1 to 7, further comprising:
valley-shaped portions (459) formed on one of the first wearing member and the second wearing member, the valley-shaped portions extending in a direction crossing the first direction and arranged along the first direction; and
mountain shaped portions (469) formed on the other of the first wearing member and the second wearing member, and the mountain shaped portions extending in a direction crossing the first direction and arranged along the first direction,
wherein when the second wearing member is coupled with the first wearing member to at least partially face the first wearing member, at least one of the mountain shaped portions is configured to be accommodated in any one of the valley shaped portions or to be engaged with any one of the valley shaped portions.

9. The wearable electronic device of any one of claims 1 to 8, further comprising:
a flexible display (420) disposed on at least one of the first wearing member or the second wearing member.

10. The wearable electronic device of claim 1, further comprising:
a processor (120; 1422); and
memory (130; 1430) configured to store instructions,
wherein the instructions are configured to, when executed by the processor, cause the wearable electronic device to:
when execution of an exercise-related function is identified in a first wearing state in which the second wearing member is moved on the first wearing member to a position for detecting a first pressure, enter a second wearing state in which the second wearing member is moved on the first wearing member to a position for detecting a second pressure higher than the first pressure; and
when termination of the execution of the exercise-related function is identified in the second wearing state, enter a third wearing state in which the second wearing member is moved on the first wearing member to a position for detecting a third pressure lower than the first pressure.

11. The wearable electronic device of claim 10, wherein the instructions are configured to, when executed by the processor, cause the electronic device to:
enter the first wearing state when a specified time has elapsed in the third wearing state.

12. The wearable electronic device of any one of claims 10 to 11, wherein the instructions are configured to, when executed by the processor, cause the electronic device to:
upon detecting the wearable electronic device is worn on a part of user's body, enter the first wearing state.

13. The wearable electronic device of any one of claims 10 to 12, wherein the instructions are configured to, when executed by the processor, cause the electronic device to:
identify a direction of movement of the second wearing member moving on the first wearing member based on first detection information received from a geomagnetic sensor.

14. The wearable electronic device of any one of claims 10 to 13, wherein the instructions are configured to, when executed by the processor, cause the electronic device to:
identify a pressure value from second detection information received from a pressure sensor;
identify a wearing state as the first wearing state when the pressure value falls within a first predetermined range for the first pressure;
identify a wearing state as the second wearing state when the pressure value falls within a second predetermined range for the second pressure; and
identify a wearing state as the third wearing state when the pressure value falls within a third predetermined range for the third pressure.

15. The wearable electronic device of any one of claims 1 to 14, the first magnetic bodies include permanent magnets and the at least one pair of second magnetic bodies include electromagnets.
